(19) Europäisches Patentamt
European Patent Office
Office européen des brevets

(11) EP 3 778 574 A1

(12) EUROPEAN PATENT APPLICATION
published in accordance with Art. 153(4) EPC

(43) Date of publication:
**17.02.2021 Bulletin 2021/07**

(21) Application number: **19782224.0**

(22) Date of filing: **03.04.2019**

(51) Int Cl.:
*C07D 231/12* (2006.01) *A01N 41/06* (2006.01)
*A01N 43/56* (2006.01) *A01N 47/04* (2006.01)
*A01P 5/00* (2006.01) *A01P 7/04* (2006.01)
*A61K 31/415* (2006.01) *A61K 31/4184* (2006.01)
*A61K 31/423* (2006.01) *A61K 31/4245* (2006.01)
*A61K 31/4439* (2006.01) *A61P 33/00* (2006.01)
*C07D 235/18* (2006.01) *C07D 249/08* (2006.01)
*C07D 263/57* (2006.01) *C07D 271/06* (2006.01)
*C07D 271/10* (2006.01) *C07D 401/04* (2006.01)
*C07D 403/04* (2006.01) *C07D 413/04* (2006.01)

(86) International application number:
**PCT/JP2019/014789**

(87) International publication number:
**WO 2019/194220 (10.10.2019 Gazette 2019/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.04.2018 JP 2018073976**

(71) Applicant: **Nippon Soda Co., Ltd.**
**Tokyo 100-8165 (JP)**

(72) Inventors:
- **KATO Tetsuro**
  **Odawara-shi, Kanagawa 250-0280 (JP)**
- **TAKAHASHI Jun**
  **Odawara-shi, Kanagawa 250-0280 (JP)**
- **SAIKI Yuto**
  **Odawara-shi, Kanagawa 250-0280 (JP)**
- **IWASA Takao**
  **Odawara-shi, Kanagawa 250-0280 (JP)**
- **SUGAHARA Erika**
  **Odawara-shi, Kanagawa 250-0280 (JP)**

(74) Representative: **Mewburn Ellis LLP**
**Aurora Building**
**Counterslip**
**Bristol BS1 6BX (GB)**

# (54) (HETERO)ARYLSULFONAMIDE COMPOUND AND PEST CONTROL AGENT

(57) A compound represented by formula (I) or a salt thereof, as well as a formulation for controlling harmful organisms, an insecticidal formulation, an acaricidal formulation, a nematicidal formulation, or a formulation for controlling or exterminating endoparasites, containing at least one compound selected from the group consisting of the compounds described above and salts thereof, as an active ingredient.

[Chem. 1]

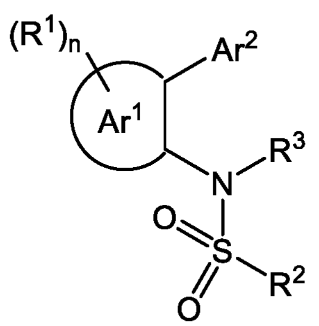

(I)

In formula (I), $Ar^1$ is a benzene ring or a 5- to 6-membered

EP 3 778 574 A1

heteroaryl ring, $R^1$ is a C1 to C6 alkyl group, a halogeno group, or the like, n represents the number of $R^1$ and is 0, 1, 2, or 3, and in the case of n being 2 or more, two or more $R^1$ may be the same as or different from one another, $R^2$ is a C1 to C6 alkyl group, a C1 to C6 haloalkyl group, or the like, $R^3$ is a hydrogen atom, a C1 to C6 alkyl group, a C1 to C6 alkylcarbonyl group, or the like, and $Ar^2$ represents a substituted or unsubstituted heteroaryl group.

**Description**

TECHNICAL FIELD

The present invention relates to a heteroaryl sulfonamide compound and a formulation for controlling harmful organisms. More specifically, the present invention relates to a (hetero)aryl sulfonamide compound which has excellent insecticidal and/or acaricidal and/or nematicidal activity, exhibits excellent safety, and can be advantageously synthesized industrially, and also relates to a formulation for controlling harmful organisms containing the same as an active ingredient.

The present application claims priority on Japanese Patent Application No. 2018-073976, filed in Japan on April 6, 2018, the content of which is incorporated herein by reference.

BACKGROUND ART

Various compounds having insecticidal and/or acaricidal activity and/or nematicidal activity have been proposed. In order to practically use such compounds as agrochemicals, the compounds are required not only to have sufficient efficacy, but also to hardly cause chemical resistance, avoid phytotoxicity against plants or soil contamination, and have a low level of toxicity against livestock, fish or the like.

A compound represented by formula (A) is disclosed in Patent Document 1. According to Patent Document 1, such compounds seem to be usuful as a parasiticide.

[Chem. 1]

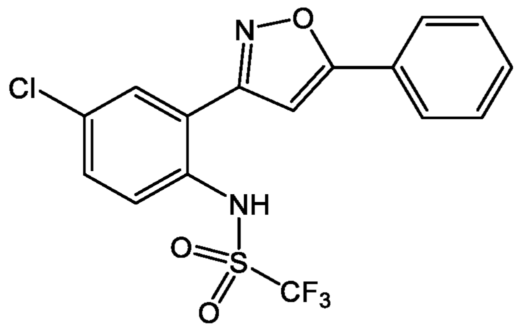

(A)

A compound represented by formula (B) is disclosed in Patent Document 2. According to Patent Document 2, such compounds seem to have an effect with respect to plant-parasitic nematodes.

[Chem. 2]

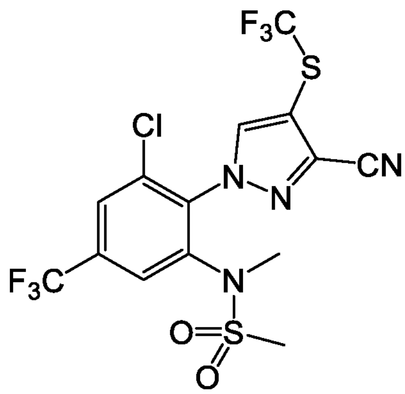

(B)

PRIOR ART LITERATURE

Patent Documents

Patent Document 1: WO 2006/034333 A1
Patent Document 2: WO 2005/090314 A1

SUMMARY OF THE INVENTION

Problems to be Solved by the Invention

Objects of the present invention are to provide a (hetero)aryl sulfonamide compound which has excellent activity for controlling harmful organisms, and in particular, has excellent insecticidal, acaricidal and/or nematicidal activity, exhibits excellent safety, and can be advantageously synthesized industrially, as well as to provide a formulation for controlling harmful organisms, the formulation containing the compound as an active ingredient.

Means for Solving the Problems

As a result of intensive studies in order to achieve the objects mentioned above, the inventors of the present application completed the present invention including the following modes.

[1] A compound represented by formula (I) or a salt thereof.

[Chem. 3]

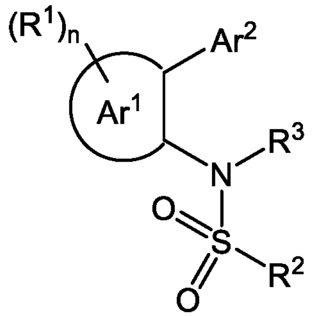

(I)

In formula (I),

$Ar^1$ is a benzene ring or a 5- to 6-membered heteroaryl ring,
$R^1$ is a substituted or unsubstituted C1 to C6 alkyl group, a substituted or unsubstituted C2 to C6 alkenyl group, a substituted or unsubstituted C2 to C6 alkynyl group, a hydroxyl group, a substituted or unsubstituted C1 to C6 alkoxy group, a formyl group, a substituted or unsubstituted C1 to C6 alkylcarbonyl group, a carboxyl group, a substituted or unsubstituted C1 to C6 alkoxycarbonyl group, a substituted or unsubstituted C1 to C6 alkylcarbonyloxy group, a mercapto group, a substituted or unsubstituted C1 to C6 alkylthio group, a substituted or unsubstituted C1 to C6 alkylsulfinyl group, a substituted or unsubstituted C1 to C6 alkylsulfonyl group, a substituted or unsubstituted C3 to C8 cycloalkyl group, a substituted or unsubstituted C6 to C10 aryl group, a substituted or unsubstituted heteroaryl group, a substituted or unsubstituted C6 to C10 aryloxy group, a substituted or unsubstituted heteroaryloxy group, a halogeno group, a nitro group, a cyano group, a group represented by $-NR^aR^b$, a group represented by $-(C=O)-NR^cR^d$, or a group represented by $-O-(C=O)-NR^cR^d$, n represents the number of $R^1$ and is 0, 1, 2, or 3, and in the case of n being 2 or more, two or more $R^1$ may be the same or different from one another,
each of $R^a$ and $R^b$ independently represents a hydrogen atom, a substituted or unsubstituted C1 to C6 alkyl group, a substituted or unsubstituted C1 to C6 alkylcarbonyl group, or a substituted or unsubstituted C1 to C6

alkoxycarbonyl group,
each of $R^c$ and $R^d$ independently represents a hydrogen atom, or a substituted or unsubstituted C1 to C6 alkyl group,
$R^2$ is a C1 to C6 alkyl group, a C1 to C6 haloalkyl group, a C3 to C8 cycloalkyl group, a C3 to C8 halocycloalkyl group, or a C3 to C8 cycloalkyl C1 to C6 alkyl group, or a C3 to C8 halocycloalkyl C1 to C6 alkyl group,
$R^3$ is a hydrogen atom, a substituted or unsubstituted C1 to C6 alkyl group, a substituted or unsubstituted C1 to C6 alkyl carbonyl group, a substituted or unsubstituted C1 to C6 alkoxy carbonyl group, a substituted or unsubstituted C1 to C6 alkylsulfonyl group, a substituted or unsubstituted C3 to C8 cycloalkyl group, a substituted or unsubstituted C3 to C8 cycloalkyl carbonyl group, or a substituted or unsubstituted C3 to C8 cycloalkoxy carbonyl group, and
$Ar^2$ represents a substituted or unsubstituted heteroaryl group.

[2] The compound according to [1] mentioned above or a salt thereof, wherein Formula (I) is Formula (II).

[Chem. 4]

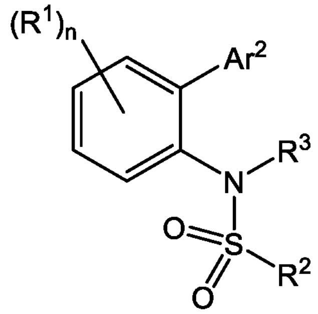

(II)

In Formula (II), $R^1$, $R^2$, $R^3$, $Ar^2$, and n represent the same meanings as those recited in Formula (I).
[3] The compound according to [1] mentioned above or a salt thereof, wherein Formula (I) is Formula (III).

[Chem. 5]

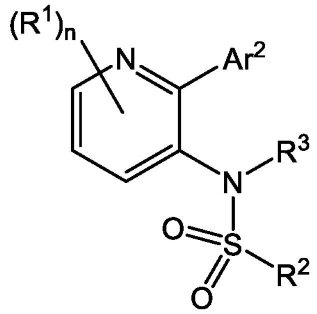

(III)

In Formula (III), $R^1$, $R^2$, $R^3$, $Ar^2$, and n represent the same meanings as those recited in Formula (I).
[4] A formulation for controlling harmful organisms, containing at least one compound selected from the group consisting of the compounds as recited in any one of [1] to [3] mentioned above and salts thereof, as an active ingredient.
[5] An insecticidal or acaricidal formulation, containing at least one compound selected from the group consisting of the compounds as recited in any one of [1] to [3] mentioned above and salts thereof, as an active ingredient.
[6] A nematicidal formulation, containing at least one compound selected from the group consisting of the compounds as recited in any one of [1] to [3] mentioned above and salts thereof, as an active ingredient.
[7] A formulation for controlling or exterminating endoparasites, containing at least one compound selected from the group consisting of the compounds as recited in any one of [1] to [3] mentioned above and salts thereof, as an active ingredient.

Effects of the Invention

The (hetero)aryl sulfonamide compound of the present invention has activity of controlling harmful organisms, and in particular, has excellent insecticidal, acaricidal and/or nematicidal activity, exhibits excellent safety, and can be advantageously synthesized industrially.

The formulation for controlling harmful organisms of the present invention can control harmful organisms which are problematic in view of farm products or for hygiene reasons. The formulation for controlling harmful organisms of the present invention exhibits excellent control effects on agriculturally harmful organisms even with a reduced concentration.

EMBODIMENTS FOR CARRYING OUT THE INVENTION

The (hetero)aryl sulfonamide compound of the present invention is a compound represented by formula (I) (hereafter also referred to as the compound (I) in some cases) or a salt of the compound (I).

[Chem. 6]

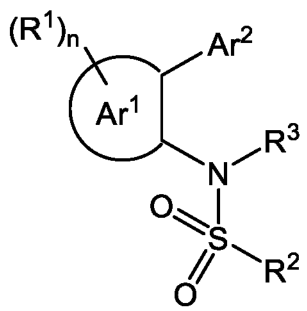

(I)

In the present invention, the term "unsubstituted" means only the core group. When the term "substituted" does not appear and only the name of the core group is recorded, the meaning "unsubstituted" is implied unless specifically stated otherwise.

On the other hand, the term "substituted" means that one of the hydrogen atoms of the core group has been substituted with a group having a structure either the same as or different from the core group. Accordingly, the "substituent" is another group that is bonded to the core group. There may be either one substituent, or two or more substituents. In the case of two or more substituents being present, the substituents may be the same or different.

There are no particular limitations on the "substituent", as long as it is chemically permissible and yields a compound having the effects of the present invention.

Specific examples of groups that can be a "substituent" include the groups listed below.

Halogeno groups such as a fluoro group, a chloro group, a bromo group, and an iodo group;

C1 to C6 alkyl groups such as a methyl group, an ethyl group, an n-propyl group, an i-propyl group, an n-butyl group, an s-butyl group, an i-butyl group, a t-butyl group, an n-pentyl group, and an n-hexyl group;

C2 to C6 alkenyl groups such as a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group;

C2 to C6 alkynyl groups such as an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group;

C3 to C8 cycloalkyl groups such as a cyclopropyl group, a cyclobutyl group, a cyclopentyl group, a cyclohexyl group, a cycloheptyl group, and a cubanyl group;

C3 to C8 cycloalkenyl groups such as a 2-cyclopropenyl group, a 2-cyclopentenyl group, a 3-cyclohexenyl group, and a 4-cyclooctenyl group;

C6 to C10 aryl groups such as a phenyl group and a naphthyl group;

5-membered heteroaryl groups such as a pyrrolyl group, a furyl group, a thienyl group, an imidazolyl group, a pyrazolyl group, an oxazolyl group, an isoxazolyl group, a thiazolyl group, an isothiazolyl group, a triazolyl group,

an oxadiazolyl group, a thiadiazolyl group, and a tetrazolyl group;
6-membered heteroaryl groups such as a pyridyl group, a pyrazinyl group, a pyrimidinyl group, a pyridazinyl group, and a triazinyl group;
condensed ring heteroaryl groups such as an indolyl group, a benzofuryl group, a benzothienyl group, a benzoimidazolyl group, a benzoxazolyl group, a benzothiazolyl group, a quinolyl group, an isoquinolyl group, and a quinoxalinyl group;
cyclic ether groups such as an oxiranyl group, a tetrahydrofuryl group, a dioxolanyl group, and a dioxanyl group;
cyclic amino groups such as an aziridinyl group, a pyrrolidinyl group, a piperidinyl group, a piperazinyl group, and a morpholinyl group;
a hydroxyl group; an oxo group;
C1 to C6 alkoxy groups such as a methoxy group, an ethoxy group, an n-propoxy group, an i-propoxy group, an n-butoxy group, an s-butoxy group, an i-butoxy group, and a t-butoxy group;
C2 to C6 alkenyloxy groups such as a vinyloxy group, an allyloxy group, a propenyloxy group, and a butenyloxy group;
C2 to C6 alkynyloxy groups such as an ethynyloxy group and a propargyloxy group;
C6 to C10 aryloxy groups such as a phenoxy group and a naphthoxy group;
5- to 6-membered ring heteroaryloxy groups such as a thiazolyloxy group and a pyridyloxy group;
a carboxyl group;
a formyl group; C1 to C6 alkylcarbonyl groups such as an acetyl group, and a propionyl group;
C1 to C6 alkylcarbonyloxy groups such as a formyloxy group, an acetyloxy group, and a propionyloxy group;
C1 to C6 alkoxycarbonyl groups such as a methoxycarbonyl group, an ethoxycarbonyl group, an n-propoxycarbonyl group, an i-propoxycarbonyl group, an n-butoxycarbonyl group, and a t-butoxycarbonyl group;
C1 to C6 haloalkyl groups such as a chloromethyl group, a chloroethyl group, a trifluoromethyl group, a 1,2-dichloro-n-propyl group, a 1-fluoro-n-butyl group, and a perfluoro-n-pentyl group;
C2 to C6 haloalkenyl groups such as a 2-chloro-1-propenyl group and a 2-fluoro-1-butenyl group;
C2 to C6 haloalkynyl groups such as a 4,4-dichloro-1-butynyl group, a 4-fluoro-1-pentynyl group, and a 5-bromo-2-pentynyl group;
C3 to C6 halocycloalkyl groups such as a 3,3-difluorocyclobutyl group;
C1 to C6 haloalkoxy groups such as a 2-chloro-n-propoxy group, a 2,3-dichlorobutoxy group, a trifluoromethoxy group, and a 2,2,2-trifluoroethoxy group;
C2 to C6 haloalkenyloxy groups such as a 2-chloropropenyloxy group and a 3-bromobutenyloxy group;
C1 to C6 haloalkylcarbonyl groups such as a chloroacetyl group, a trifluoroacetyl group, and a trichloroacetyl group;
halogeno-substituted phenyl groups such as a fluorophenyl group, a difluorophenyl group, a chlorophenyl group, a dichlorophenyl group, a bromophenyl group, a dibromophenyl group, an iodophenyl group, and a diiodophenyl group;
a cyano group; a nitro group; an amino group;
C1 to C6 alkylamino groups such as a methylamino group, a dimethylamino group, and a diethylamino group;
C6 to C10 arylamino groups such as an anilino group and a naphthylamino group;
a formylamino group; C1 to C6 alkylcarbonylamino groups such as an acetylamino group, a propanoylamino group, a butyrylamino group, and an i-propylcarbonylamino group;
C1 to C6 alkoxycarbonylamino groups such as a methoxycarbonylamino group, an ethoxycarbonylamino group, an n-propoxycarbonylamino group, and an i-propoxycarbonylamino group;
C1 to C6 alkylsulfoxyimino groups such as an S,S-dimethylsulfoxyimino group;
an aminocarbonyl group;
C1 to C6 alkylaminocarbonyl groups such as a methylaminocarbonyl group, a dimethylaminocarbonyl group, an ethylaminocarbonyl group, and an i-propylaminocarbonyl group;
imino C1 to C6 alkyl groups such as an iminomethyl group, a (1-imino)ethyl group, and a (1-imino)-n-propyl group;
C1 to C6 alkoxyaminocarbonyl groups such as a methoxyaminocarbonyl group, an ethoxyaminocarbonyl group, and an i-propoxyaminocarbonyl group;
hydroxyimino C1 to C6 alkyl groups such as a hydroxyiminomethyl group, a (1-hydroxyimino)ethyl group, and a (1-hydroxyimino)propyl group;
C1 to C6 alkoxyimino C1 to C6 alkyl groups such as a methoxyiminomethyl group and a (1-methoxyimino)ethyl group;
a mercapto group;
a pentafluorosulfanyl group;
C1 to C6 alkylthio groups such as a methylthio group, an ethylthio group, an n-propylthio group, an i-propylthio group, an n-butylthio group, an i-butylthio group, an s-butylthio group, and a t-butylthio group;
C1 to C6 haloalkylthio groups such as a trifluoromethylthio group and a 2,2,2-trifluoroethylthio group;
C2 to C6 alkenylthio groups such as a vinylthio group and an allylthio group;
C2 to C6 alkynylthio groups such as an ethynylthio group and a propargylthio group;
C1 to C6 alkylsulfinyl groups such as a methylsulfinyl group, an ethylsulfinyl group, and a t-butylsulfinyl group;

C1 to C6 haloalkylsulfinyl groups such as a trifluoromethylsulfinyl group and a 2,2,2-trifluoroethylsulfinyl group;
C2 to C6 alkenylsulfinyl groups such as an allylsulfinyl group;
C2 to C6 alkynylsulfinyl groups such as a propargylsulfinyl group;
C1 to C6 alkylsulfonyl groups such as a methylsulfonyl group, an ethylsulfonyl group, and a t-butylsulfonyl group;
C1 to C6 haloalkylsulfonyl groups such as a trifluoromethylsulfonyl group and a 2,2,2-trifluoroethylsulfonyl group;
C2 to C6 alkenylsulfonyl groups such as an allylsulfonyl group;
C2 to C6 alkynylsulfonyl groups such as a propargylsulfonyl group;
tri C1 to C6 alkylsilyl groups such as a trimethylsilyl group, a triethylsilyl group, and a t-butyldimethylsilyl group; and
tri C6 to C10 arylsilyl groups such as a triphenylsilyl group.

In addition, in these "substituents", any of the hydrogen atoms in any of the above substituents may be substituted with a group of a different structure. Examples of the "substituents" in such cases include C1 to C6 alkyl groups, C1 to C6 haloalkyl groups, C1 to C6 alkoxy groups, C1 to C6 haloalkoxy groups, halogeno groups, a cyano group and a nitro group.

Terms such as "C1 to C6" indicate that the number of carbon atoms in the core group ranges from 1 to 6 or the like. This number of carbon atoms does not include the number of carbon atoms that exist within substituents. For example, in the case of an ethoxybutyl group, the core group is a butyl group and an ethoxy group is a substituent, and therefore this group is classified as a C2 alkoxy C4 alkyl group.

In Formula (I), $Ar^1$ is a benzene ring or a 5- to 6-membered heteroaryl ring.

Examples of the "5- to 6-membered heteroaryl ring" for $Ar^1$ include 5-membered heteroaryl rings such as a pyrrole ring, a furan ring, a thiophene ring, an imidazole ring, a pyrazole ring, an oxazole ring, an isoxazole ring, a thiazole ring, and an isothiazole ring; and 6-membered heteroaryl groups such as a pyridine ring, a pyrazine ring, a pyrimidine ring, and a pyridazine ring.

The group represented by $-NR^3S(=O)_2R^2$ and the $Ar^2$ described below are bonded at the ortho position on the $Ar^1$.

$Ar^1$ is preferably a benzene ring, a pyridine ring, a pyrimidine ring, or pyridazine ring, and is more preferably a benzene ring or a pyridine ring.

In Formula (I), $R^1$ is a substituted or unsubstituted C1 to C6 alkyl group, a substituted or unsubstituted C2 to C6 alkenyl group, a substituted or unsubstituted C2 to C6 alkynyl group, a hydroxyl group, a substituted or unsubstituted C1 to C6 alkoxy group, a formyl group, a substituted or unsubstituted C1 to C6 alkyl carbonyl group, a carboxyl group, a substituted or unsubstituted C1 to C6 alkoxy carbonyl group, a substituted or unsubstituted C1 to C6 alkyl carbonyloxy group, a mercapto group, a substituted or unsubstituted C1 to C6 alkylthio group, a substituted or unsubstituted C1 to C6 alkylsulfinyl group, a substituted or unsubstituted C1 to C6 alkylsulfonyl group, a substituted or unsubstituted C3 to C8 cycloalkyl group, a substituted or unsubstituted C6 to C10 aryl group, a substituted or unsubstituted heteroaryl group, a substituted or unsubstituted C6 to C10 aryloxy group, a substituted or unsubstituted heteroaryloxy group, a halogeno group, a nitro group, a cyano group, a group represented by $-NR^aR^b$, a group represented by $-(C=O)-NR^cR^d$, or a group represented by $-O-(C=O)-NR^cR^d$.

The "C1 to C6 alkyl group" for $R^1$ may be linear or branched. Examples of the alkyl group include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group, and an i-hexyl group.

Examples of the preferred substituents on the "C1 to C6 alkyl group" of $R^1$ include halogeno groups such as a fluoro group, a chloro group, a bromo group, and an iodo group; C1 to C6 alkoxy groups such as a methoxy group, an ethoxy group, an n-propoxy group, an i-propoxy group, an n-butoxy group, an s-butoxy group, an i-butoxy group, and a t-butoxy group; C1 to C6 haloalkoxy groups such as a 2-chloro-n-propoxy group, a 2,3-dichlorobutoxy group, and a trifluoromethoxy group; and a cyano group.

Examples of the preferred "substituted C1 to C6 alkyl group" include C1 to C6 haloalkyl groups such as a chloromethyl group, a chloroethyl group, a trifluoromethyl group, a pentafluoroethyl group, a 1,2-dichloro-n-propyl group, a 1,1,1,3,3,3-hexafluoropropan-2-yl group, a perfluoropropan-2-yl group, a 1-fluoro-n-butyl group, and a perfluoro-n-pentyl group.

Examples of the "C2 to C6 alkenyl group" for $R^1$ include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group and a 5-hexenyl group.

Examples of the "C2 to C6 alkynyl group" for $R^1$ include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

Examples of the "C1 to C6 alkoxy group" for $R^1$ include a methoxy group, an ethoxy group, an n-propoxy group, an n-butoxy group, an n-pentyloxy group, an n-hexyloxy group, an i-propoxy group, an i-butoxy group, an s-butoxy group, a t-butoxy group, and an i-hexyloxy group.

Examples of the "C1 to C6 alkylcarbonyl group" for $R^1$ include an acetyl group and a propionyl group.

Examples of the "C1 to C6 alkoxycarbonyl group" for $R^1$ include a methoxycarbonyl group, an ethoxycarbonyl group, an n-propoxycarbonyl group, an i-propoxycarbonyl group, and a t-butoxycarbonyl group.

Examples of the "C1 to C6 alkylcarbonyloxy group" for $R^1$ include an acetyloxy group, a propionyloxy group, and a butyryloxy group.

Examples of the "C1 to C6 alkylthio group" for $R^1$ include a methylthio group, an ethylthio group, an n-propylthio group, an n-butylthio group, an n-pentylthio group, an n-hexylthio group, and an i-propylthio group.

Examples of the "C1 to C6 alkylsulfinyl group" for $R^1$ include a methylsulfinyl group, an ethylsulfinyl group, and a t-butylsulfinyl group.

Examples of the "C1 to C6 alkylsulfonyl group" for $R^1$ include a methylsulfonyl group, an ethylsulfonyl group, and a t-butylsulfonyl group.

Examples of preferred substituents on the "C2 to C6 alkenyl group", "C2 to C6 alkynyl group", "C1 to C6 alkoxy group", "C1 to C6 alkylcarbonyl group", "C1 to C6 alkoxycarbonyl group", "C1 to C6 alkylthio group", "C1 to C6 alkylsulfinyl group", "C1 to C6 alkylsulfonyl group", and "C1 to C6 alkylcarbonyloxy group" for $R^1$ include halogeno groups such as a fluoro group, a chloro group, a bromo group, and an iodo group; C1 to C6 alkoxy groups such as a methoxy group, an ethoxy group, an n-propoxy group, an i-propoxy group, an n-butoxy group, an s-butoxy group, an i-butoxy group, and a t-butoxy group; C1 to C6 haloalkoxy groups such as a 2-chloro-n-propoxy group, a 2,3-dichlorobutoxy group, and a trifluoromethoxy group; and a cyano group.

Examples of the "C3 to C8 cycloalkyl group" for $R^1$ include a cyclopropyl group, a cyclobutyl group, a cyclopentyl group, a cyclohexyl group, and a cycloheptyl group.

The "C6 to C10 aryl group" for $R^1$ may be a monocyclic aryl group or a polycyclic aryl group. In a polycyclic group, provided at least one ring is an aromatic ring, each remaining ring may be a saturated alicyclic ring, an unsaturated alicyclic ring or an aromatic ring.

Examples of the "C6 to C10 aryl group" include a phenyl group, a naphthyl group, an azulenyl group, an indenyl group, an indanyl group, and a tetralinyl group.

Examples of the "heteroaryl group" for $R^1$ include 5-membered heteroaryl groups such as a pyrrolyl group, a furyl group, a thienyl group, an imidazolyl group, a pyrazolyl group, an oxazolyl group, an isoxazolyl group, a thiazolyl group, an isothiazolyl group, a triazolyl group (and specifically, a [1,2,3]-triazolyl group or a [1,2,4]-triazolyl group), an oxadiazolyl group (and specifically, a [1,2,3]-oxadiazolyl group, a [1,2,4]-oxadiazolyl group, a [1,2,5]-oxadiazolyl group or a [1,3,4]-oxadiazolyl group), a thiadiazolyl group, and a tetrazolyl group; 6-membered heteroaryl groups such as a pyridyl group, a pyrazinyl group, a pyrimidinyl group, a pyridazinyl group, and a triazinyl group; and condensed ring heteroaryl groups such as an indolyl group, a benzofuryl group, a benzothienyl group, a benzoimidazolyl group, a benzoxazolyl group, a benzothiazolyl group, a quinolyl group, an isoquinolyl group, and a quinoxalinyl group.

Examples of the "C6 to C10 aryloxy group" for $R^1$ include a phenoxy group, a naphthyloxy group, an azulenyloxy group, an indenyloxy group, an indanyloxy group, and a tetralinyloxy group.

Examples of the "heteroaryloxy group" for $R^1$ include 5-and 6-membered ring heteroaryloxy groups such as a thiazolyloxy group and a pyridyloxy group.

Examples of preferred substituents on the "C3 to C8 cycloalkyl group", "C6 to C10 aryl group", "heteroaryl group", "C6 to C10 aryloxy group" and "heteroaryloxy group" for $R^1$ include halogeno groups such as a fluoro group, a chloro group, a bromo group, and an iodo group; and C1 to C6 haloalkyl groups such as a chloromethyl group, a chloroethyl group, a trifluoromethyl group, a pentafluoroethyl group, a 1,2-dichloro-n-propyl group, a 1,1,1,3,3,3-hexafluoropropan-2-yl group, a perfluoropropan-2-yl group, a 1-fluoro-n-butyl group, and a perfluoro-n-pentyl group; and the like.

Examples of the "halogeno group" for $R^1$ include a fluoro group, a chloro group, a bromo group, an iodo group, and the like.

Each of $R^a$ and $R^b$ in the "group represented by $-NR^aR^b$" for $R^1$ is independently a hydrogen atom, a substituted or unsubstituted C1 to C6 alkyl group, a substituted or unsubstituted C1 to C6 alkylcarbonyl group, or a substituted or unsubstituted C1 to C6 alkoxycarbonyl group.

Examples of the "substituted or unsubstituted C1 to C6 alkyl group", "substituted or unsubstituted C1 to C6 alkylcarbonyl group" and "substituted or unsubstituted C1 to C6 alkoxycarbonyl group" for $R^a$ and $R^b$ in the "group represented by $-NR^aR^b$" include the same groups as those exemplified in $R^1$.

Each of $R^c$ and $R^d$ in the "group represented by $-(C=O)-NR^cR^d$" and the "group represented by $-O-(C=O)-NR^cR^d$" for $R^1$ is independently a hydrogen atom, or a substituted or unsubstituted C1 to C6 alkyl group.

Examples of the "substituted or unsubstituted C1 to C6 alkyl group" for $R^c$ and $R^d$ in the "group represented by $-(C=O)-NR^cR^d$" and the "group represented by $-O-(C=O)-NR^cR^d$" for $R^1$ include the same groups as those exemplified

in $R^1$.

$R^c$ and $R^d$ are preferably a hydrogen atom.

$R^1$ is preferably a halogeno group, a C1 to C6 alkyl group, a C1 to C6 haloalkyl group, a substituted or unsubstituted C1 to C6 alkoxy group, a substituted or unsubstituted C1 to C6 alkylthio group, a substituted or unsubstituted C6 to C10 aryl group, a substituted or unsubstituted heteroaryl group, a group represented by -(C=O)-$NR^cR^d$, or a cyano group, and is more preferably a halogeno group, a C1 to C6 haloalkyl group, a substituted or unsubstituted C6 to C10 aryl group, or a group represented by - (C=O)-$NR^cR^d$.

In formula (I), n represents the number of $R^1$, and is an integer of any one of 0 to 3. In the case of n being 2 or more, two or more $R^1$ may be the same or different from one another.

n is preferably 0 to 1, and is more preferably 1.

In formula (I), $R^2$ is a C1 to C6 alkyl group, a C1 to C6 haloalkyl group, a C3 to C8 cycloalkyl group, a C3 to C8 halocycloalkyl group, or a C3 to C8 cycloalkyl C1 to C6 alkyl group, or a C3 to C8 halocycloalkyl C1 to C6 alkyl group.

Examples of the "C1 to C6 alkyl group" and "C3 to C8 cycloalkyl group" for $R^2$ include the same groups as those exemplified in $R^1$.

Examples of the "C1 to C6 haloalkyl group" for $R^2$ include a fluoromethyl group, a chloromethyl group, a bromomethyl group, an iodomethyl group, a difluoromethyl group, a dichloromethyl group, a trifluoromethyl group, a chlorodifluoromethyl group, a trichloromethyl group, a bromodifluoromethyl group, a 2-fluoroethyl group, a 2-chloroethyl group, a 2-bromoethyl group, a 2,2-difluoroethyl group, a 2,2,2-trifluoroethyl group, a 2-chloro-2,2-difluoroethyl group, a 2,2,2-trichloroethyl group, a 1,1,2,2-tetrafluoroethyl group, a 2-chloro-1,1,2-trifluoroethyl group, a pentafluoroethyl group, a 2,2-difluoropropyl group, a 3,3,3-trifluoropropyl group, a 2,2,3,3,3-pentafluoropropyl group, a 1,1,2,3,3,3-hexafluoropropyl group, a heptafluoropropyl group, a 2,2,2-trifluoro-1-(trifluoromethyl)ethyl group, a 1,2,2,2-tetrafluoro-1-(trifluoromethyl)ethyl group, a 2,2,3,3,4,4,4-heptafluorobutyl group, and a nonafluorobutyl group.

Examples of the "C3 to C8 halocycloalkyl group" for $R^2$ include a 1-fluorocyclopropyl group, a 2-fluorocyclopropyl group, a 1-chlorocyclopropyl group, a 2-chlorocyclopropyl group, a 2,2-difluorocyclopropyl group, a 2,2-dichlorocyclopropyl group, a 2,2,3,3-tetrafluorocyclopropyl group, a 2-fluorocyclopentyl group, a 3-fluorocyclopentyl group, a 2-chlorocyclopentyl group, a 3-chlorocyclopentyl group, a 3,4-difluorocyclohexyl group, a 3,4-dichlorocyclohexyl group, and a 3,4-dibromocyclohexyl group.

Examples of the "C3 to C8 cycloalkyl C1 to C6 alkyl group" for $R^2$ include a cyclopropylmethyl group and the like.

Examples of the "C3 to C8 halocycloalkyl C1 to C6 alkyl group" for $R^2$ include a 2-fluorocyclopropylmethyl group, a 1-fluorocyclopropylmethyl group, a 1,2-difluorocyclopropylmethyl group, and the like.

$R^2$ is preferably a C1 to C6 alkyl group or a C1 to C6 haloalkyl group, is more preferably a C1 to C6 fluoroalkyl group such as a fluoromethyl group, a difluoromethyl group, a trifluoromethyl group, a 2-fluoroethyl group, a 2,2-difluoroethyl group, a 2,2,2-trifluoroethyl group, a 1,1,2,2-tetrafluoroethyl group, a pentafluoroethyl group, a 2,2-difluoropropyl group, a 3,3,3-trifluoropropyl group, a 2,2,3,3,3-pentafluoropropyl group, a 1,1,2,3,3,3-hexafluoropropyl group, a heptafluoropropyl group, a 2,2,2-trifluoro-1-(trifluoromethyl)ethyl group, a 1,2,2,2-tetrafluoro-1-(trifluoromethyl)ethyl group, a 2,2,3,3,4,4,4-heptafluorobutyl group, or a nonafluorobutyl group, and is in particular, preferably a trifluoromethyl group, a 2,2,2-trifluoroethyl group, or a 2,2-difluoropropyl group.

In formula (I), $R^3$ is a hydrogen atom, a substituted or unsubstituted C1 to C6 alkyl group, a substituted or unsubstituted C1 to C6 alkylcarbonyl group, a substituted or unsubstituted C1 to C6 alkoxycarbonyl group, a substituted or unsubstituted C1 to C6 alkylsulfonyl group, a substituted or unsubstituted C3 to C8 cycloalkyl group, a substituted or unsubstituted C3 to C8 cycloalkylcarbonyl group, or a substituted or unsubstituted C3 to C8 cycloalkoxycarbonyl group.

Examples of the "substituted or unsubstituted C1 to C6 alkyl group", "substituted or unsubstituted C1 to C6 alkylcarbonyl group", "substituted or unsubstituted C1 to C6 alkoxycarbonyl group", "substituted or unsubstituted C1 to C6 alkylsulfonyl group", and "substituted or unsubstituted C3 to C8 cycloalkyl group" for $R^3$ include the same groups as those exemplified in $R^1$.

Examples of the "C3 to C8 cycloalkylcarbonyl group" for $R^3$ include a cyclopropanecarbonyl group, a cyclopentanecarbonyl group, and a cyclohexanecarbonyl group.

Examples of the "C3 to C8 cycloalkoxycarbonyl group" for $R^3$ include a cyclopropoxycarbonyl group, a cyclopentyloxycarbonyl group, and a cyclohexyloxycarbonyl group.

Examples of preferred substituents on the "C3 to C8 cycloalkylcarbonyl group" and "C3 to C8 cycloalkoxycarbonyl group" for $R^3$ include halogeno groups such as a fluoro group, a chloro group, a bromo group, and iodo group; C1 to C6 alkyl groups such as a methyl group, an ethyl group, an n-propyl group, an i-propyl group, an n-butyl group, an s-butyl group, an i-butyl group, a t-butyl group, an n-pentyl group, and an n-hexyl group; and C1 to C6 haloalkyl groups such as a chloromethyl group, a chloroethyl group, a trifluoromethyl group, a 1,2-dichloro-n-propyl group, a 1-fluoro-n-butyl group, and a perfluoro-n-pentyl group.

$R^3$ is preferably a hydrogen atom, or a substituted or unsubstituted C1 to C6 alkylsulfonyl group, and is more preferably a hydrogen atom.

In formula (I), $Ar^2$ is a substituted or unsubstituted heteroaryl group.

Examples of the "heteroaryl group" for $Ar^2$ include the same groups as those exemplified above for $R^1$.

Examples of preferred substituents on the "heteroaryl group" for $Ar^2$ include halogeno groups such as a fluoro group, a chloro group, a bromo group, and an iodo group; C1 to C6 alkyl groups such as a methyl group, an ethyl group, an n-propyl group, an i-propyl group, an n-butyl group, an s-butyl group, an i-butyl group, a t-butyl group, an n-pentyl group, and an n-hexyl group; C1 to C6 haloalkyl groups such as a chloromethyl group, a chloroethyl group, a trifluoromethyl group, a 2,2,2-trifluoroethyl group, a pentafluoroethyl group, a 1,2-dichloro-n-propyl group, a 1,1,1,3,3,3-hexafluoropropan-2-yl group, a perfluoropropan-2-yl group, a 1-fluoro-n-butyl group, and a perfluoro-n-pentyl group; C1 to C6 alkoxycarbonyl groups such as a methoxycarbonyl group, an ethoxycarbonyl group, an n-propoxycarbonyl group, an i-propoxycarbonyl group, an n-butoxycarbonyl group, and a t-butoxycarbonyl group; C3 to C8 cycloalkyl groups such as a cyclopropyl group, a cyclobutyl group, a cyclopentyl group, a cyclohexyl group, a cycloheptyl group, and a cubanyl group; halogeno-substituted phenyl groups such as a 3,4-difluorophenyl group, a 4-chlorophenyl group, and a 4-bromophenyl group; C1 to C6 haloalkyl-substituted phenyl groups such as a 4-(trifluoromethyl)phenyl group; 5-membered heteroaryl groups such as a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group; an amino group; and C1 to C6 alkylaminocarbonyl groups such as a methylaminocarbonyl group, a dimethylaminocarbonyl group, an ethylaminocarbonyl group, and an i-propylaminocarbonyl group. Examples of more preferable substituents on the "heteroaryl group" for $Ar^2$ include a halogeno group; a C1 to C6 alkyl group; a C1 to C6 haloalkyl group; a C1 to C6 alkoxycarbonyl group; a halogeno-substituted phenyl group; a C1 to C6 haloalkyl-substituted phenyl group. Examples of even more preferable substituents thereon include a halogeno group; a C1 to C6 fluoroalkyl group such as a trifluoromethyl group, or a 2,2,2-trifluoroethyl group; a halogeno-substituted phenyl group; and a C1 to C6 haloalkyl-substituted phenyl group. Examples of particularly preferable substituents thereon include a halogeno group; and a C1 to C6 fluoroalkyl group.

$Ar^2$ is preferably a substituted or unsubstituted pyrazolyl group, and is more preferably a substituted pyrazolyl group.

$Ar^2$ is preferably a substituted or unsubstituted triazole group.

$Ar^2$ is preferably a substituted or unsubstituted imidazole group.

$Ar^2$ is preferably a substituted or unsubstituted oxadiazole group.

$Ar^2$ is preferably a substituted or unsubstituted pyridyl group.

$Ar^2$ is preferably a substituted or unsubstituted benzimidazolyl group.

$Ar^2$ is preferably a substituted or unsubstituted benzoxazolyl group.

There are no particular limitations on the salt of the compound (I), as long as the salt is an agriculturally and horticulturally acceptable salt. Examples thereof include salts of inorganic acids such as hydrochloric acid and sulfuric acid; salts of organic acids such as acetic acid and lactic acid; salts of alkali metals such as lithium, sodium and potassium; salts of alkaline earth metals such as calcium and magnesium; salts of transition metals such as iron and copper; and salts of organic bases such as ammonia, triethylamine, tributylamine, pyridine and hydrazine.

The compound (I) or the salt of the compound (I) is not particularly limited by the production method thereof. In addition, the salt of the compound (I) can be obtained from the compound (I) by a known method. The compound (I) or the salt of the compound (I) of the present invention can be obtained by a known preparation method. For example, the compound (I) or the salt of the compound (I) of the present invention can be obtained by a known production method described in Examples and the like.

[Compound represented by Formula (II)]

The (hetero)aryl sulphonamide compound of the present invention is preferably a compound represented by Formula (II).

[Chem. 7]

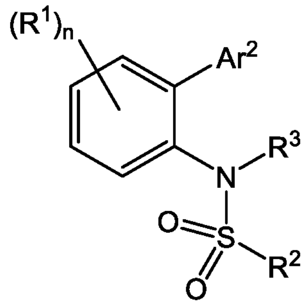

(II)

In Formula (II), $R^1$, $R^2$, $R^3$, $Ar^2$, and n represent the same meanings as those recited in Formula (I).

[Compound represented by Formula (III)]

The (hetero)aryl sulphonamide compound of the present invention is preferably a compound represented by Formula (III).

[Chem. 8]

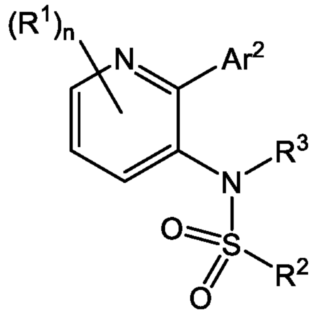

(III)

In Formula (III), $R^1$, $R^2$, $R^3$, $Ar^2$, and n represent the same meanings as those recited in Formula (I).

[Compound represented by Formula (IV)]

The (hetero)aryl sulphonamide compound of the present invention is preferably a compound represented by Formula (IV).

[Chem. 9]

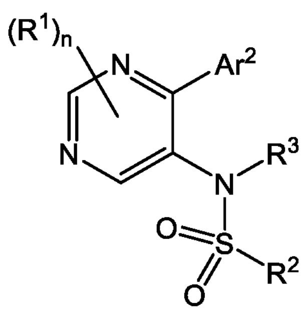

(IV)

In Formula (IV), $R^1$, $R^2$, $R^3$, $Ar^2$, and n represent the same meanings as those recited in Formula (I).

[Compound represented by Formula (V)]

The (hetero)aryl sulphonamide compound of the present invention is preferably a compound represented by Formula (V).

[Chem. 10]

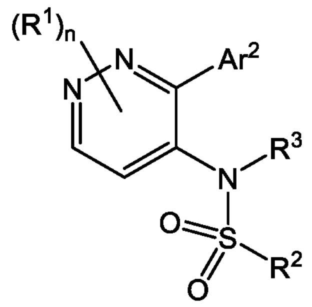

(V)

In Formula (V), $R^1$, $R^2$, $R^3$, $Ar^2$, and n represent the same meanings as those recited in Formula (I).

[Compound represented by Formula (VI)]

The (hetero)aryl sulphonamide compound of the present invention is preferably a compound represented by Formula (VI).

[Chem. 11]

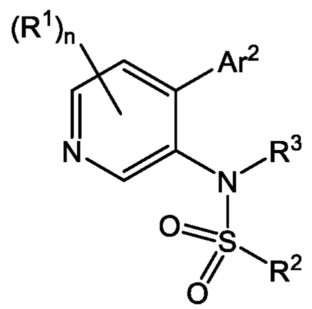

(VI)

In Formula (VI), $R^1$, $R^2$, $R^3$, $Ar^2$, and n represent the same meanings as those recited in Formula (I).

The (hetero)aryl sulfonamide compounds of the present invention have excellent effects of controlling harmful organisms such as various agriculturally harmful organisms, acari and nematodes that affect the growth of plants.

In addition, the (hetero)aryl sulfonamide compounds of the present invention cause no chemical damage to crops and exhibit low toxicity to fish and warm-blooded animals, and for this reason, they are very safe compounds. For this reason, the compounds are useful as an active ingredient for insecticidal formulations, acaricidal formulations and nematicidal formulations.

Moreover, in recent years, many harmful insects such as Plutellidae, Delphacidae, Cicadellidae and Aphididae have developed resistance to various known chemicals, causing problems of inadequate potency for these chemicals, and there is much demand for a chemical that is also effective against these resistant strains. The (hetero)aryl sulfonamide compounds of the present invention exhibit excellent effects for controlling harmful insects of not only sensitive strains but also various resistant strains, and controlling acari of acaricide-resistant strains.

In addition, the (hetero)aryl sulfonamide compounds of the present invention exhibit efficacy against all growth stages of the control target organisms, and for example, exhibit a control effect on the eggs, nymphs, larvae, pupae and adults of acari, insects and nematodes.

[Formulation for controlling harmful organisms]

The formulation for controlling harmful organisms of the present invention contains at least one compound selected from the compounds of the present invention as an active ingredient. There are no particular limitations on the amount of the compound of the present invention contained within the formulation for controlling harmful organisms of the present invention, as long as an effect of controlling harmful organisms is exhibited. Examples of the formulation for controlling harmful organisms of the present invention include an insecticidal or acaricidal formulation, a nematicidal formulation, and a formulation for controlling endoparasites or expelling endoparasites.

[Insecticidal, acaricidal, or nematicidal formulation]

The insecticidal, acaricidal or nematicidal formulation of the present invention contains at least one compound selected from the (hetero)aryl sulfonamide compounds of the present invention as an active ingredient. There are no particular limitations on the amount of the (hetero)aryl sulfonamide compound of the present invention contained within the insecticidal, acaricidal or nematicidal formulation of the present invention, as long as an effect of controlling harmful organisms is exhibited.

The insecticidal, acaricidal or nematicidal formulation of the present invention is preferably used on plants such as grains; vegetables; root vegetables; tubers; flowers and ornamental plants; fruit trees; trees such as foliage plants, tea plants, coffee plants, and cacao plants; pasture grasses; lawn grasses; and cotton plants.

During application to the plant, the formulation for controlling harmful organisms, or the insecticidal, acaricidal or nematicidal formulation of the present invention may be applied to any portion of the plant, including the leaves, stems, stalks, flowers, buds, fruit, seeds, sprouts, roots, tubers, tuberous roots, shoots or cuttings.

Furthermore, the formulation for controlling harmful organisms, or the insecticidal, acaricidal or nematicidal formulation of the present invention is not particularly limited in terms of the types of plants on which it can be applied. Examples of the types of plants include original species, variants, improved varieties, cultivars, mutants, hybrids, and genetically modified species (GMO).

The insecticidal, acaricidal or nematicidal formulation of the present invention can be used for a seed treatment, foliage application, soil application, or water surface application, for the purpose of controlling various agriculturally harmful insects, acari and nematodes.

Specific examples of the various agriculturally harmful insects, acari and nematodes that can be controlled by the formulation for controlling harmful organisms of the present invention are listed below.

(1) Lepidoptera Butterflies and Moths

(a) Arctiidae moths, for example, Hyphantria cunea and Lemyra imparilis;
(b) Bucculatricidae moths, for example, Bucculatrix pyrivorella;
(c) Carposinidae, for example, Carposina sasakii;
(d) Crambidae moths, for example, Diaphania indica and Diaphania nitidalis of Diaphania spp.; Ostrinia furnacalis, Ostrinia nubilalis and Ostrinia scapulalis of Ostrinia spp.; and others such as Chilo suppressalis, Cnaphalocrocis medinalis, Conogethes punctiferalis, Diatraea grandiosella, Glyphodes pyloalis, Hellula undalis and Parapediasia teterrella;
(e) Gelechiidae moths, for example, Helcystogramma triannulella, Pectinophora gossypiella, Phthorimaea operculella and Sitotroga cerealella;
(f) Geometridae moths, for example, Ascotis selenaria;
(g) Gracillariidae moths, for example, Caloptilia theivora, Phyllocnistis citrella and Phyllonorycter ringoniella;
(h) Hesperiidae butterflies, for example, Parnara guttata;
(i) Lasiocampidae moths, for example, Malacosoma neustria;
(j) Lymantriidae moths, for example, Lymantria dispar and Lymantria monacha of Lymantria spp.; and others such as Euproctis pseudoconspersa and Orgyia thyellina;
(k) Lyonetiidae moths, for example, Lyonetia clerkella and Lyonetia prunifoliella malinella of Lyonetia spp.;
(l) Noctuidae moths, for example, Spodoptera depravata, Spodoptera eridania, Spodoptera exigua, Spodoptera frugiperda, Spodoptera littoralis and Spodoptera litura of Spodoptera spp.; Autographa gamma and Autographa nigrisigna of Autographa spp.; Agrotis ipsilon and Agrotis segetum of Agrotis spp.; Helicoverpa armigera, Helicoverpa assulta and Helicoverpa zea of Helicoverpa spp.; Heliothis armigera and Heliothis virescens of Heliothis spp.; and others such as Aedia leucomelas, Ctenoplusia agnata, Eudocima tyrannus, Mamestra brassicae, Mythimna separata, Naranga aenescens, Panolis japonica, Peridroma saucia, Pseudoplusia includens and Trichoplusia ni;
(m) Nolidae moths, for example, Earias insulana;

(n) Pieridae butterflies, for example, Pieris brassicae and Pieris rapae crucivora of Pieris spp.;
(o) Plutellidae moths, for example, Acrolepiopsis sapporensis and Acrolepiopsis suzukiella of Acrolepiopsis spp.; and others such as Plutella xylostella;
(p) Pyralidae moths, for example, Cadra cautella, Elasmopalpus lignosellus, Etiella zinckenella and Galleria mellonella;
(q) Sphingidae moths, for example, Manduca quinquemaculata and Manduca sexta of Manduca spp.;
(r) Stathmopodidae moths, for example, Stathmopoda masinissa;
(s) Tineidae moths, for example, Tinea translucens;
(t) Tortricidae moths, for example, Adoxophyes honmai and Adoxophyes orana of Adoxophyes spp.; Archips breviplicanus and Archips fuscocupreanus of Archips spp.; and others such as Choristoneura fumiferana, Cydia pomonella, Eupoecilia ambiguella, Grapholitha molesta, Homona magnanima, Leguminivora glycinivorella, Lobesia botrana, Matsumuraeses phaseoli, Pandemis heparana and Sparganothis pilleriana; and
(u) Yponomeutidae moths, for example, Argyresthia conjugella.

(2) Thysanoptera Harmful Insects

(a) Phlaeothripidae, for example, Ponticulothrips diospyrosi; and
(b) Thripidae, for example, Frankliniella intonsa and Frankliniella occidentalis of Frankliniella spp.; Thrips palmi and Thrips tabaci of Thrips spp.; and others such as Heliothrips haemorrhoidalis and Scirtothrips dorsalis.

(3) Hemiptera Harmful Insects

(A) Archaeorrhyncha

(a) Delphacidae, for example, Laodelphax striatella, Nilaparvata lugens, Perkinsiella saccharicida and Sogatella furcifera.

(B) Clypeorrhyncha

(a) Cicadellidae, for example, Empoasca fabae, Empoasca nipponica, Empoasca onukii and Empoasca sakaii of Empoasca spp.; and others such as Arboridia apicalis, Balclutha saltuella, Epiacanthus stramineus, Macrosteles striifrons and Nephotettix cinctinceps.

(C) Heteroptera

(a) Alydidae, for example, Riptortus clavatus;
(b) Coreidae, for example, Cletus punctiger and Leptocorisa chinensis;
(c) Lygaeidae, for example, Blissus leucopterus, Cavelerius saccharivorus and Togo hemipterus;
(d) Miridae, for example, Halticus insularis, Lygus lineolaris, Psuedatomoscelis seriatus, Stenodema sibiricum, Stenotus rubrovittatus and Trigonotylus caelestialium;
(e) Pentatomidae, for example, Nezara antennata and Nezara viridula of Nezara spp.; Eysarcoris aeneus, Eysarcoris lewisi and Eysarcoris ventralis of Eysarcoris spp.; and others such as Dolycoris baccarum, Eurydema rugosum, Glaucias subpunctatus, Halyomorpha halys, Piezodorus hybneri, Plautia crossota and Scotinophora lurida;
(f) Pyrrhocoridae, for example, Dysdercus cingulatus;
(g) Rhopalidae, for example, Rhopalus msculatus;
(h) Scutelleridae, for example, Eurygaster integriceps; and
(i) Tingidae, for example, Stephanitis nashi.

(D) Sternorrhyncha

(a) Adelgidae, for example, Adelges laricis;
(b) Aleyrodidae, for example, Bemisia argentifolii and Bemisia tabaci of Bemisia spp.; and others such as Aleurocanthus spiniferus, Dialeurodes citri and Trialeurodes vaporariorum;
(c) Aphididae, for example, Aphis craccivora, Aphis fabae, Aphis forbesi, Aphis gossypii, Aphis pomi, Aphis sambuci and Aphis spiraecola of Aphis spp.; Rhopalosiphum maidis and Rhopalosiphum padi of Rhopalosiphum spp.; Dysaphis plantaginea and Dysaphis radicola of Dysaphis spp.; Macrosiphum avenae and Macrosiphum euphorbiae of Macrosiphum spp.; Myzus cerasi, Myzus persicae and Myzus varians of Myzus

spp.; and others such as Acyrthosiphon pisum, Aulacorthum solani, Brachycaudus helichrysi, Brevicoryne brassicae, Chaetosiphon fragaefolii, Hyalopterus pruni, Hyperomyzus lactucae, Lipaphis erysimi, Megoura viciae, Metopolophium dirhodum, Nasonovia ribis-nigri, Phorodon humuli, Schizaphis graminum, Sitobion avenae and Toxoptera aurantii;
(d) Coccidae, for example, Ceroplastes ceriferus and Ceroplastes rubens of Ceroplastes spp.;
(e) Diaspididae, for example, Pseudaulacaspis pentagona and Pseudaulacaspis prunicola of Pseudaulacaspis spp.; Unaspis euonymi and Unaspis yanonensis of Unaspis spp.; and others such as Aonidiella aurantii, Comstockaspis perniciosa, Fiorinia theae and Pseudaonidia paeoniae;
(f) Margarodidae, for example, Drosicha corpulenta and Icerya purchasi;
(g) Phylloxeridae, for example, Viteus vitifolii;
(h) Pseudococcidae, for example, Planococcus citri and Planococcus kuraunhiae of Planococcus spp.; and others such as Phenacoccus solani and Pseudococcus comstocki; and
(i) Psyllidae, for example, Psylla mali and Psylla pyrisuga of Psylla spp.; and others such as Diaphorina citri.

(4) Polyphaga harmful insects

(a) Anobiidae, for example, Lasioderma serricorne;
(b) Attelabidae, for example, Byctiscus betulae and Rhynchites heros;
(c) Bostrichidae, for example, Lyctus brunneus;
(d) Brentidae, for example, Cylas formicarius;
(e) Buprestidae, for example, Agrilus sinuatus;
(f) Cerambycidae, for example, Anoplophora malasiaca, Monochamus alternatus, Psacothea hilaris and Xylotrechus pyrrhoderus;
(g) Chrysomelidae, for example, Bruchus pisorum and Bruchus rufimanus of Bruchus spp.; Diabrotica barberi, Diabrotica undecimpunctata and Diabrotica virgifera of Diabrotica spp.; Phyllotreta nemorum and Phyllotreta striolata of Phyllotreta spp.; and others such as Aulacophora femoralis, Callosobruchus chinensis, Cassida nebulosa, Chaetocnema concinna, Leptinotarsa decemlineata, Oulema oryzae and Psylliodes angusticollis;
(h) Coccinellidae, for example, Epilachna varivestis and Epilachna vigintioctopunctata of Epilachna spp.;
(i) Curculionidae, for example, Anthonomus grandis and Anthonomus pomorum of Anthonomus spp.; Sitophilus granarius of Sitophilus zeamais of Sitophilus spp.; and others such as Echinocnemus squameus, Euscepes postfasciatus, Hylobius abietis, Hypera postica, Lissohoptrus oryzophilus, Otiorhynchus sulcatus, Sitona lineatus and Sphenophorus venatus;
(j) Elateridae, for example, Melanotus fortnumi and Melanotus tamsuyensis of Melanotus spp.;
(k) Nitidulidae, for example, Epuraea domina;
(l) Scarabaeidae, for example, Anomala cuprea and Anomala rufocuprea of Anomala spp.; and others such as Cetonia aurata, Gametis jucunda, Heptophylla picea, Melolontha melolontha and Popillia japonica;
(m) Scolytidae, for example, Ips typographus;
(n) Staphylinidae, for example, Paederus fuscipes;
(o) Tenebrionidae, for example, Tenebrio molitor and Tribolium castaneum; and
(p) Trogossitidae, for example, Tenebroides mauritanicus.

(5) Diptera harmful insects

(A) Brachycera

(a) Agromyzidae, for example, Liriomyza bryoniae, Liriomyza chinensis, Liriomyza sativae and Liriomyza trifolii of Liriomyza spp.; and others such as Chromatomyia horticola and Agromyza oryzae;
(b) Anthomyiidae, for example, Delia platura and Delia radicum of Delia spp.; and others such as Pegomya cunicularia;
(c) Drosophilidae, for example, Drosophila melanogaster and Drosophila suzukii of Drosophila spp.;
(d) Ephydridae, for example, Hydrellia griseola;
(e) Psilidae, for example, Psila rosae; and
(f) Tephritidae, for example, Bactrocera cucurbitae and Bactrocera dorsalis of Bactrocera spp.; Rhagoletis cerasi and Rhagoletis pomonella of Rhagoletis spp.; and others such as Ceratitis capitata and Dacus oleae.

(B) Nematocera

(a) Cecidomyiidae, for example, Asphondylia yushimai, Contarinia sorghicola, Mayetiola destructor and

Sitodiplosis mosellana.

(6) Orthoptera harmful insects

(a) Acrididae, for example, Schistocerca americana and Schistocerca gregaria of Schistocerca spp.; and others such as Chortoicetes terminifera, Dociostaurus maroccanus, Locusta migratoria, Locustana pardalina, Nomadacris septemfasciata and Oxya yezoensis;
(b) Gryllidae, for example, Acheta domestica and Teleogryllus emma;
(c) Gryllotalpidae, for example, Gryllotalpa orientalis; and
(d) Tettigoniidae, for example, Tachycines asynamorus.

(7) Acari

(A) Acaridida of Astigmata

(a) Acaridae mites, for example, Rhizoglyphus echinopus and Rhizoglyphus robini of Rhizoglyphus spp.; Tyrophagus neiswanderi, Tyrophagus perniciosus, Tyrophagus putrescentiae and Tyrophagus similis of Tyrophagus spp.; and others such as Acarus siro, Aleuroglyphus ovatus and Mycetoglyphus fungivorus;

(B) Actinedida of Prostigmata

(a) Tetranychidae mites, for example, Bryobia praetiosa and Bryobia rubrioculus of Bryobia spp.; Eotetranychus asiaticus, Eotetranychus boreus, Eotetranychus celtis, Eotetranychus geniculatus, Eotetranychus kankitus, Eotetranychus pruni, Eotetranychus shii, Eotetranychus smithi, Eotetranychus suginamensis and Eotetranychus uncatus of Eotetranychus spp.; Oligonychus hondoensis, Oligonychus ilicis, Oligonychus karamatus, Oligonychus mangiferus, Oligonychus orthius, Oligonychus perseae, Oligonychus pustulosus, Oligonychus shinkajii and Oligonychus ununguis of Oligonychus spp.; Panonychus citri, Panonychus mori and Panonychus ulmi of Panonychus spp.; Tetranychus cinnabarinus, Tetranychus kanzawai, Tetranychus ludeni, Tetranychus quercivorus, Tetranychus phaselus, Tetranychus urticae and Tetranychus viennensis of Tetranychus spp.; Aponychus corpuzae and Aponychus firmianae of Aponychus spp.; Sasanychus akitanus and Sasanychus pusillus of Sasanychus spp.; Shizotetranychus celarius, Shizotetranychus longus, Shizotetranychus miscanthi, Shizotetranychus recki and Shizotetranychus schizopus of Shizotetranychus spp.; and others such as Tetranychina harti, Tuckerella pavoniformis and Yezonychus sapporensis;
(b) Tenuipalpidae mites, for example, Brevipalpus lewisi, Brevipalpus obovatus, Brevipalpus phoenicis, Brevipalpus russulus and Brevipalpus californicus of Brevipalpus spp.; Tenuipalpus pacificus and Tenuipalpus zhizhilashviliae of Tenuipalpus spp.; and others such as Dolichotetranychus floridanus;
(c) Eriophyidae mites, for example, Aceria diospyri, Aceria ficus, Aceria japonica, Aceria kuko, Aceria paradianthi, Aceria tiyingi, Aceria tulipae and Aceria zoysiea of Aceria spp.; Eriophyes chibaensis and Eriophyes emarginatae of Eriophyes spp.; Aculops lycopersici and Aculops pelekassi of Aculops spp.; Aculus fockeui and Aculus schlechtendali of Aculus spp.; and others such as Acaphylla theavagrans, Calacarus carinatus, Colomerus vitis, Calepitrimerus vitis, Epitrimerus pyri, Paraphytoptus kikus, Paracalacarus podocarpi and Phyllocotruta citri;
(d) Transonemidae mites, for example, Tarsonemus bilobatus and Tarsonemus waitei of Tarsonemus spp.; and others such as Phytonemus pallidus and Polyphagotarsonemus latus; and
(e) Penthaleidae mites, for example, Penthaleus erythrocephalus and Penthaleus major of Penthaleus spp.

(8) Phytoparasitic Nematodes

(A) Tylenchida

(a) Anguinidae, for example, Anguina funesta and Anguina tritici of Anguina spp.; and Ditylenchus destructor, Ditylenchus dipsaci and Ditylenchus myceliophagus of Ditylenchus spp.;
(b) Aphelenchoididae, for example, Aphelenchoides besseyi, Aphelenchoides fragariae, Aphelenchoides ritzemabosi and Aphelenchoides besseyi of Aphelenchoides spp.; and Bursaphelenchus xylophilus of Bursaphelenchus spp.;
(c) Belonolaimidae, for example, Belonolaimus longicaudatus of Belonolaimus spp.; and Tylenchorhynchus claytoni and Tylenchorhynchus dubius of Tylenchorhynchus spp.;
(d) Criconematidae, for example, Criconema mutabile;

(e) Dolichodoridae, for example, Dolichodorus mediterraneus;
(f) Ecphyadophoridae, for example, Ecphyadophora tenuissima;
(g) Hemicycliophoridae, for example, Loofia thienemanni
(h) Heteroderidae, for example, Globodera rostochiensis, Globodera pallida and Globodera tabacum of Globodera spp.; and Heterodera avenae, Heterodera cruciferae, Heterodera glycines, Heterodera schachtii and Heterodera trifolii of Heterodera spp.;
(i) Hoplolaimidae, for example, Helicotylenchus dihystera and Helicotylenchus multicinctus of Helicotylenchus spp.; Hoplolaimus columbus and Hoplolaimus galeatus of Hoplolaimus spp.; and others such as Rotylenchus robustus and Rotylenchulus reniformis;
(j) Meloidogynidae, for example, Meloidogyne arenaria, Meloidogyne chitwoodi, Meloidogyne hapla, Meloidogyne incognita, Meloidogyne javanica and Meloidogyne thamesi of Meloidogyne spp.;
(k) Nothotylenchidae, for example, Nothotylenchus acris;
(l) Paratylenchidae, for example, Paratylenchus curvitatus and Paratylenchus elachistus of Paratylenchus spp.; and
(m) Pratylenchidae, for example, Pratylenchus brachyurus, Pratylenchus coffeae, Pratylenchus curvitatus, Pratylenchus fallax, Pratylenchus goodeyi, Pratylencus neglectus, Pratylenchus penetrans, Pratylenchus scribneri, Pratylenchus vulnus and Pratylenchus zeae of Pratylenchus spp.; and others such as Nacobbus aberrans, Radopholus similis, Tylenchulus semipenetrans and Radopholus citrophilus.

(B) Dorylaimida

(a) Longidoridae, for example, Longidorus elongates of Longidorus spp.; and Xiphinema americanum, Xiphinema brevicolle, Xiphinema index and Xiphinema diversicaudatum of Xiphinema spp.

(C) Triplonchida

(a) Trichodoridae, for example, Trichodorus primitivus and Paratrichodorus minor.

The formulation for controlling harmful organisms of the present invention may be mixed or used in combination with other active constituents such as fungicides, insecticides and acaricides, nematicides and soil insecticides; and/or plant regulators, herbicides, synergists, fertilizers, soil conditioners and animal feed.

Combinations of the active ingredient seleced from the compounds of the present invention with other active constituents can be expected to provide synergistic effects in terms of insecticidal, acaricidal and nematicidal activity. Such a synergistic effect can be confirmed using the Colby equation in accordance with typical methods (Colby, S.R.; Calculating Synergistic and Antagonistic Responses of Herbicide Combinations; Weeds 15, pp. 20 to 22, 1967).

Specific examples of insecticides, acaricides, nematicides, soil pesticides, and parasiticides and the like that can be mixed or used in combination with the formulation for controlling harmful organisms of the present invention are listed below.

(1) Acetylcholinesterase inhibitors:

(a) Carbamate-based: alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, oxamyl, pirimicarb, propoxur, thiodicarb, thiofanox, triazamate, trimethacarb, XMC, xylycarb, fenothiocarb, MIPC, MPMC, MTMC, aldoxycarb, allyxycarb, aminocarb, bufencarb, cloethocarb, metam-sodium, and promecarb; and
(b) Organophosphorus-based: acephate, azamethiphos, azinphos-ethyl, azinphos-methyl, cadusafos, chlorethoxyfos, chlorfenvinphos, chlormephos, chlorpyrifos, chlorpyrifos-methyl, coumaphos, cyanophos, demeton-S-methyl, diazinon, dichlorvos/DDVP, dicrotophos, dimethoate, dimethylvinfos, disulfoton, EPN, ethion, ethoprophos, famphur, fenamiphos, fenitrothion, fenthion, fosthiazate, heptenophos, imicyafos, isofenphos, isocarbophos, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridafenthion, quinalphos, sulfotep, tebupirimfos, temephos, terbufos, tetrachlorvinphos, thiometon, triazophos, trichlorfon, vamidothion; bromophos-ethyl, BRP, carbophenothion, cyanofenphos, CYAP, demeton-S-methyl sulfone, dialifos, dichlofenthion, dioxabenzofos, etrimfos, fensulfothion, flupyrazofos, fonofos, formothion, fosmethilan, isazophos, iodofenphos, methacrifos, pirimiphos-ethyl, phosphocarb, propaphos, prothoate, and sulprofos.

(2) GABA receptor chloride ion channel antagonists: acetoprole, chlordane, endosulfan, ethiprole, fipronil, pyrafluprole, pyriprole; camphechlor, heptachlor, and dienochlor.

(3) Sodium channel modulators: acrinathrin, d-cis/trans-allethrin, d-trans-allethrin, bifenthrin, bioallethrin, bioallethrin S-cyclopentyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans isomer], delta-methrin, empenthrin [(EZ)-(1R)-isomer], esfenvalerate, ethofenprox, fenpropathrin, fenvalerate, flucythrinate, flumethrin, tau-fluvalinate, halfenprox, imiprothrin, kadethrin, permethrin, phenothrin [(1R)-trans isomer], prallethrin, pyrethrum, resmethrin, silafluofen, tefluthrin, tetramethrin [(1R)-isomer], tralomethrin, transfluthrin; allethrin, pyrethrin, pyrethrin I, pyrethrin II, profluthrin, dimefluthrin, bioethanomethrin, biopermethrin, transpermethirn, fenfluthrin, fenpyrithrin, flubrocythrinate, flufenoprox, metofluthrin, protrifenbute, pyresmethrin, and terallethrin.

(4) Nicotinic acetylcholine receptor agonists: acetamiprid, clothianidin, dinotefuran, imidacloprid, nitenpyram, nithiazine, thiacloprid, thiamethoxam, sulfoxaflor, nicotine, and flupyradifurone.

(5) Nicotinic acetylcholine receptor allosteric modulators: spinetoram and spinosad.

(6) Chloride channel activators: abamectin, emamectin-benzoate, lepimectin, milbemectin; ivermectin, seramectin, doramectin, eprinomectin, moxidectin, milbemycin, milbemycin oxime, and nemadectin.

(7) Juvenile hormone-like substances: hydroprene, kinoprene, methoprene, fenoxycarb, pyriproxyfen; diofenolan, epofenonane, and triprene.

(8) Other nonspecific inhibitors: methyl bromide, chloropicrin, sulfuryl fluoride, borax, and tartar emetic.

(9) Homoptera selective feeding inhibitors: flonicamid, pymetrozine, and pyrifluquinazon.

(10) Acari growth inhibitors: clofentezine, diflovidazin, hexythiazox, and etoxazole.

(11) Microorganism-derived insect midgut inner membrane disrupting agents: Bacillus thuringiensis subsp. israelensis, Bacillus sphaericus, Bacillus thuringiensis subsp. aizawai, Bacillus thuringiensis subsp. kurstaki, Bacillus thuringiensis subsp. tenebrionis, Bt crop protein: Cry1Ab, Cry1Ac, Cry1Fa, Cry1A.105, Cry2Ab, Vip3A, mCry3A, Cry3Ab, Cry3Bb, Cry34Ab1/Cry35Ab1.

(12) Mitochondria ATP biosynthesis enzyme inhibitors: diafenthiuron, azocyclotin, cyhexatin, fenbutatin oxide, propargite, and tetradifon.

(13) Oxidative phosphorylation uncoupling agents: chlorfenapyr, sulfluramid, DNOC; binapacryl, dinobuton, and dinocap.

(14) Nicotinic acetylcholine receptor channel blockers: bensultap, cartap hydrochloride, nereistoxin, thiosultap-sodium, and thiocyclarm.

(15) Chitin synthesis inhibitors: bistrifluron, chlorfluazuron, diflubenzuron, flucycloxuron, flufenoxuron, hexaflumuron, lufenuron, novaluron, novifumuron, teflubenzuron, triflumuron, buprofezin, and fluazuron.

(16) Diptera molting disturbing agent: cyromazine.

(17) Molting hormone receptor agonists: chromafenozide, halofenozide, methoxyfenozide, and tebufenozide.

(18) Octopamine receptor agonists: amitraz, demiditraz, and chlordimeform

(19) Mitochondrial electron transport chain complex III inhibitors: acequinocyl, fluacrypyrim, and hydramethylnon.

(20) Mitochondrial electron transport chain complex I inhibitors: fenazaquin, fenproximate, pyrimidifen, pyridaben, tebufenpyrad, tolfenpyrad, and rotenone.

(21) Voltage-dependent sodium channel blockers: indoxacarb and metaflumizone.

(22) Acetyl CoA carboxylase inhibitors: spirodiclofen, spiromesifen, and spirotetramat.

(23) Mitochondrial electron transport chain complex IV inhibitors: aluminium phosphide, calcium phosphide, phosphine, zinc phosphide, and cyanide.

(24) Mitochondrial electron transport chain complex II inhibitors: cyenopyrafen, cyflumetofen, and pyflubumide.

(25) Ryanodine receptor modulators: chlorantraniliprole, cyantraniliprole, flubendiamide, cyclaniliprole, and tetraniliprole.

(26) Mixed function oxidase inhibitor compound: piperonyl butoxide.

(27) Latrophilin receptor agonists: depsipeptide, cyclodepsipeptide, 24-membered cyclodepsipeptide, emodepside.

(28) Others (for which the mode of action is unknown): azadirachtin, benzoximate, bifenazate, bromopropylate, quinomethionate, cryolite, dicofol, pyridalyl; benclothiaz, sulfur, amidoflumet, 1,3-dichloropropene, DCIP, phenisobromolate, benzomate, metaldehyde, chlorobenzilate, chlothiazoben, dicyclanil, fenoxacrim, fentrifanil, flubenzimine, fluphenazine, gossyplure, japonilure, metoxadiazone, oil, potassium oleate, tetrasul, triarathene; afidopyropen, flometoquin, flufiprole, fluensulfone, meperfluthrin, tetramethylfluthrin, tralopyril, dimefluthrin, methylneodecanamide; fluralaner, afoxolaner, fluxametamide,5-[5-(3,5-dichlorophenyl)-5-trifluoromethyl-4,5-dihydroisoxazol-3-yl]-2-(1H-1,2,4-triazol-1-yl)benzonitrile (CAS:943137-49-3), broflanilide, and other meta-diamides.

(29) Parasiticides:

(a) Benzimidazole-based: fenbendazole, albendazole, triclabendazole, oxibendazole, mebendazole, oxfendazole, parbendazole, flubendazole; febantel, netobimin, thiophanate; thiabendazole, and cambendazole;
(b) Salicylanilide-based: closantel, oxyclozanide, rafoxanide, and niclosamide;
(c) Substituted phenol-based: nitroxinil, nitroscanate;
(d) Pyrimidine-based: pyrantel and morantel;
(e) Imidazothiazole-based: levamisole and tetramisole;
(f) Tetrahydropyrimidine-based: praziquantel and epsiprantel; and
(g) Other parasiticides: cyclodiene, ryania, clorsulon, metronidazole, demiditraz; piperazine, diethylcarbamazine, dichlorophen, monepantel, tribendimidine, amidantel; thiacetarsamide, melarsomine, and arsenamide.

Specific examples of fungicides that can be mixed or used in combination with the formulation for controlling harmful organisms of the present invention are listed below.

(1) Nucleic acid biosynthesis inhibitors:

(a) RNA polymerase I inhibitors: benalaxyl, benalaxyl-M, furalaxyl, metalaxyl, metalaxyl-M; oxadixyl; clozylacon, and ofurace;
(b) Adenosine deaminase inhibitors: bupirimate, dimethirimol, and ethirimol;
(c) DNA/RNA synthesis inhibitors: hymexazol and octhilinone; and
(d) DNA topoisomerase II inhibitor: oxolinic acid.

(2) Mitotic inhibitors and cell division inhibitors:

(a) β-tubulin polymerization inhibitors: benomyl, carbendazim, chlorfenazole, fuberidazole, thiabendazole; thiophanate, thiophanate-methyl; diethofencarb; zoxamide; and ethaboxam;
(b) Cell division inhibitor: pencycuron; and
(c) Delocalization inhibitor of spectrin-like proteins: fluopicolide.

(3) Respiration inhibitors:

(a) Complex INADH oxidoreductase inhibitors: diflumetorim; and tolfenpyrad;
(b) Complex II succinic acid dehydrogenase inhibitors: benodanil, flutolanil, mepronil; isofetamid; fluopyram; fenfuram, furmecyclox; carboxin, oxycarboxin; thifluzamide; benzovindiflupyr, bixafen, fluxapyroxad, furametpyr, isopyrazam, penflufen, penthiopyrad, sedaxane; and boscalid;
(c) Complex III ubiquinol oxidase Qo inhibitors: azoxystrobin, coumoxystrobin, coumethoxystrobin, enoxastrobin, flufenoxystrobin, picoxystrobin; pyraoxystrobin; pyraclostrobin, pyrametostrobin, triclopyricarb; kresoxim-methyl, trifloxystrobin; dimoxystrobin, fenaminstrobin, metominostrobin, orysastrobin; famoxadone; fluoxastrobin; fenamidone; and pyribencarb;
(d) Complex III ubiquinol reductase Qi inhibitors: cyazofamid; and amisulbrom;
(e) Oxidative phosphorylation uncoupling agents: binapacryl, meptyldinocap, dinocap; fluazinam,; and ferimzone;
(f) Oxidative phosphorylation inhibitors (ATP synthase inhibitors): fentin acetate, fentin chloride, and fentin hydroxide;
(g) ATP production inhibitor: silthiofam; and
(h) Complex III cytochrome bcl (ubiquinone reductase) Qx (unknown) inhibitor: ametoctradin;

(4) Amino acid and protein synthesis inhibitors

(a) Methionine biosynthesis inhibitors: andoprim, cyprodinil, mepanipyrim, and pyrimethanil; and
(b) Protein synthesis inhibitors: blasticidin-S; kasugamycin, kasugamycin hydrochloride; streptomycin; and oxytetracycline.

(5) Signal transduction inhibitors:

(a) Signal transduction inhibitors: quinoxyfen and proquinazid; and
(b) MAP/histidine kinase inhibitors in osmotic pressure signal transduction: fenpiclonil, fludioxonil; chlozolinate, iprodione, procymidone, and vinclozolin.

(6) Lipid and cell membrane synthesis inhibitors:

(a) Phospholipid biosynthesis and methyltransferase inhibitors: edifenphos, iprobenfos, pyrazophos; and isoprothiolane;
(b) Lipid peroxidation agents: biphenyl, chloroneb, dichloran, quintozene, tecnazene, tolclofos-methyl; and etridiazole;
(c) Agents that act upon cell membranes: iodocarb, propamocarb, propamocarb-hydrochloride, propamocarb-fosetylate, and prothiocarb;
(d) Microorganisms that disturb pathogen cell membranes: Bacillus subtilis, Bacillus subtilis strain QST713, Bacillus subtilis strain FZB24, Bacillus subtilis strain MBI600, and Bacillus subtilis strain D747; and
(e) Agents that disturb cell membranes: Melaleuca alternifolia (tea tree) extract.

(7) Cell membrane sterol biosynthesis inhibitors:

(a) C14 position demethylation inhibitors in sterol biosynthesis: triforine; pyrifenox, pyrisoxazole; fenarimol, flurprimidol, nuarimol; imazalil, imazalil-sulfate, oxpoconazole, pefurazoate, prochloraz, triflumizole, viniconazole; azaconazole, bitertanol, bromuconazole, cyproconazole, diclobutrazol, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, penconazole, propiconazole, quinconazole, simeconazole, tebuconazole, tetraconazole, triadimefon, triadimenol, triticonazole; prothioconazole, and voriconazole;
(b) $\Delta$14 reductase and $\Delta 8 \rightarrow \Delta 7$-isomerase inhibitors in sterol biosynthesis: aldimorph, dodemorph, dodemorph acetate, fenpropimorph, tridemorph; fenpropidin, piperalin; and spiroxamine;
(c) 3-keto reductase inhibitors in C4-position demethylation in sterol biosynthesis systems: fenhexamid; and fenpyrazamine; and
(d) Squalene epoxidase inhibitors in sterol biosynthesis systems: pyributicarb; naftifene, and terbinafine.

(8) Cell wall synthesis inhibitors:

(a) Trehalase inhibitor: validamycin;
(b) Chitin synthase inhibitors: polyoxins and polyoxorim; and
(c) Cellulose synthase inhibitors: dimethomorph, flumorph, pyrimorph; benthiavalicarb, iprovalicarb, tolprocarb, valifenalate; and mandipropamide.

(9) Melanin biosynthesis inhibitors:

(a) Reductase inhibitors in melanin biosynthesis: fthalide; pyroquilon; and tricyclazole; and
(b) Anhydrase inhibitors in melanin biosynthesis: carpropamid; diclocymet; and fenoxanil.

(10) Host plant resistance-inducing agents:

(a) Agent that acts on salicylic acid biosynthetic pathway: acibenzolar-S-methyl; and
(b) Others: probenazole; tiadinil; isotianil; laminarin; and Reynoutria sachalinensis extract.

(11) Agents for which the mode of activity is unclear: cymoxanil, fosetyl-aluminum, phosphoric acid (phosphate), tecloftalam, triazoxide, flusulfamide, diclomezine, methasulfocarb, cyflufenamid, metrafenone, pyriofenone, dodine, dodine free base, and flutianil.
(12) Agents having multiple activities: copper (copper salts), bordeaux mixture, copper hydroxide, copper naphthalate, copper oxide, copper oxychloride, copper sulfate, sulfur, sulfur products, calcium polysulfide; ferbam, mancozeb, maneb, mancopper, metiram, polycarbamate, propineb, thiram, zineb, ziram; captan, captafol, folpet; chlorothalonil; dichlofluanid, tolylfluanid; guazatine, iminoctadine triacetate, iminoctadine trialbesilate, anilazine; dithianon; quinomethionate; and fluoroimide.
(13) Other agents: DBEDC, fluorofolpet, guazatine acetate, bis(8-quinolinolato) copper (II), propamidine, chloropicrin, cyprofuram, agrobacterium, bethoxazin, diphenylamine, methyl isothiocyanate (MITC), mildiomycin, capsaicin, curfraneb, cyprosulfamide, dazomet, debacarb, dichlorophen, difenzoquat, difenzoquat methyl sulfonate, flumetover, fosetyl-calcium, fosetyl-sodium, irumamycin, natamycin, nitrothal-isopropyl, oxamocarb, puropamocin sodium, pyrrolnitrin, tebufloquin, tolnifanide, zarilamide, Algophase, Amicarthiazol, Oxathiapiprolin, metiram-zinc, benthiazole, trichlamide, uniconazole, mildiomycin, Oxyfenthiin, and picarbutrazox.

Specific examples of plant growth regulators that can be mixed or used in combination with the formulation for controlling harmful organisms of the present invention are listed below.

Abscisic acid, kinetin, benzylaminopurine, 1,3-diphenylurea, forchlorfenuron, thidiazuron, chlorfenuron, dihydrozeatin, gibberellin A, gibberellin A4, gibberellin A7, gibberellin A3, 1-methylcyclopropane, N-acetyl aminoethoxyvinylglycine (alternative name: aviglycine), aminooxyacetate, silver nitrate, cobalt chloride, IAA, 4-CPA, cloprop, 2,4-D, MCPB, indole-3-butyric acid, dichlorprop, phenothiol, 1-naphthylacetamide, ethychlozate, cloxyfonac, maleic acid hydrazide, 2,3,5-triiodobenzoic acid, salicylic acid, methyl salicylate, (-)-jasmonic acid, methyl jasmonate, (+)-strigol, (+)-deoxystrigol, (+)-orobanchol, (+)-sorgolactone, 4-oxo-4-(2-phenylethyl) aminobutyric acid; ethephon, chlormequat, mepiquat chloride, benzyl adenine, and 5-aminolevulinic acid.

[Endoparasite-Controlling Formulation or Parasiticidal Formulation]

An endoparasite-controlling formulation or parasiticidal formulation of the present invention contains at least one compound selected from the (hetero)aryl sulfonamide compounds of the present invention as an active ingredient.

The parasites targeted by the endoparasite control agent or parasiticide of the present invention live inside the bodies of host animals, and particularly inside the bodies of warm-blooded animals and fish (namely, endoparasites). Examples of host animals for which the endoparasite control agent or parasiticide of the present invention is effective include warm-blooded animals such as humans, domestic mammals (for example, cows, horses, pigs, sheep, and goats and the like), experimental animals (for example, mice, rats, and gerbils and the like), pet animals (for example, hamsters, guinea pigs, dogs, cats, horses, squirrels, rabbits, and ferrets and the like), wild mammals and zoo mammals (for example, monkeys, foxes, deer, and buffalo and the like), domestic fowl (for example, turkeys, ducks, chickens, and quail and the like), pet birds (for example, pigeons, parrots, myna birds, Java finches, parakeets, Bengalese finches, and canaries and the like); and fish such as salmon, trout, and carp and the like. By controlling or eliminating the parasites, parasitic diseases carried by the parasites can be prevented or treated.

Examples of parasites that can be controlled or eliminated include those listed below.

(1) Dioctophymatida nematodes

(a) Kidney worms of the Dioctophymatidae family, for example, Dioctophyma renale of Dioctophyma spp.; and
(b) Kidney worms of the Soboliphymatidae family, for example, Soboliphyme abei and Soboliphyme baturini of Soboliphyme spp.

(2) Trichocephalida nematodes

(a) Trichina worms of the Trichinellidae family, for example, Trichinella spiralis of Trichinella spp.; and
(b) Whipworms of the Trichuridae family, for example, Capillaria annulata, Capillaria contorta, Capillaria hepatica, Capillaria perforans, Capillaria plica and Capillaria suis of Capillaria spp.; and Trichuris vulpis, Trichuris discolor, Trichuris ovis, Trichuris skrjabini and Trichuris suis of Trichuris spp.

(3) Rhabditida nematodes
Threadworms of the Strongyloididae family, for example, Strongyloides papillosus, Strongyloides planiceps, Strongyloides ransomi, Strongyloides suis, Strongyloides stercoralis, Strongyloides tumefaciens and Strongyloides ratti of Strongyloides spp.
(4) Strongylida nematodes
Hookworms of the Ancylostomatidae family, for example, Ancylostoma braziliense, Ancylostoma caninum, Ancylostoma duodenale and Ancylostoma tubaeforme of Ancylostoma spp.; Uncinaria stenocephala of Uncinaria spp.; and Bunostomum phlebotomum and Bunostomum trigonocephalum of Bunostomum spp.
(5) Strongylida nematodes

(a) Nematodes of the Angiostrongylidae family, for example, Aelurostrongylus abstrusus of Aelurostrongylus spp.; and Angiostrongylus vasorum and Angiostrongylus cantonesis of Angiostrongylus spp.;
(b) Nematodes of the Crenosomatidae family, for example, Crenosoma aerophila and Crenosoma vulpis of Crenosoma spp.;
(c) Nematodes of the Filaroididae family, for example, Filaroides hirthi and Filaroides osleri of Filaroides spp.;
(d) Lungworms of the Metastrongylidae family, for example, Metastrongylus apri, Metastrongylus asymmetricus, Metastrongylus pudendotectus and Metastrongylus salmi of Metastrongylus spp.; and
(e) Gapeworms of the Syngamidae family, for example, Cyathostoma bronchialis of Cyathostoma spp.; and Syngamus skrjabinomorpha and Syngamus trachea of Syngamus spp.

(6) Strongylida nematodes

(a) Nematodes of the Molineidae family, for example, Nematodirus filicollis and Nematodirus spathiger of Nematodirus spp.;
(b) Nematodes of the Dictyocaulidae family, for example, Dictyocaulus filarial and Dictyocaulus viviparus of Dictyocaulus spp.;
(c) Nematodes of the Haemonchidae family, for example, Haemonchus contortus of Haemonchus spp.; and Mecistocirrus digitatus of Mecistocirrus spp.;
(d) Nematodes of the Haemonchidae family, for example, Ostertagia ostertagi of Ostertagia spp.;
(e) Nematodes of the Heligmonellidae family, for example, Nippostrongylus braziliensis of Nippostrongylus spp.; and
(f) Nematodes of the Trichostrongylidae family, for example, Trichostrongylus axei, Trichostrongylus colubriformis and Trichostrongylus tenuis of Trichostrongylus spp.; Hyostrongylus rubidus of Hyostrongylus spp.; and Obeliscoides cuniculi of Obeliscoides spp.

(7) Strongylida nematodes

(a) Nematodes of the Chabertiidae family, for example, Chabertia ovina of Chabertia spp.; and Oesophagostomum brevicaudatum, Oesophagostomum columbianum, Oesophagostomum dentatum, Oesophagostomum georgianum, Oesophagostomum maplestonei, Oesophagostomum quadrispinulatum, Oesophagostomum radiatum, Oesophagostomum venulosum and Oesophagostomum watanabei of Oesophagostomum spp.;
(b) Nematodes of the Stephanuridae family, for example, Stephanurus dentatus of Stephanurus spp.; and
(c) Nematodes of the Strongylidae family, for example, Strongylus asini, Strongylus edentatus, Strongylus equinus and Strongylus vulgaris of Strongylus spp.

(8) Oxyurida nematodes
Nematodes of the Oxyuridae family, for example, Enterobius anthropopitheci and Enterobius vermicularis of Enterobius spp.; Oxyuris equi of Oxyuris spp.; and Passalurus ambiguus of Passalurus spp.
(9) Ascaridida nematodes

(a) Nematodes of the Ascaridiidae family, for example, Ascaridia galli of Ascaridia spp.;
(b) Nematodes of the Heterakidae family, for example, Heterakis beramporia, Heterakis brevispiculum, Heterakis gallinarum, Heterakis pusilla and Heterakis putaustralis of Heterakis spp.;
(c) Nematodes of the Anisakidae family, for example, Anisakis simplex of Anisakis spp.;
(d) Nematodes of the Ascarididae family, for example, Ascaris lumbricoides and Ascaris suum of Ascaris spp.; and Parascaris equorum of Parascaris spp.; and
(e) Nematodes of the Toxocaridae family, for example, Toxocara canis, Toxocara leonina, Toxocarasuum, Toxocara vitulorum and Toxocara cati of Toxocara spp.

(10) Spirurida nematodes

(a) Nematodes of the Onchocercidae family, for example, Brugia malayi, Brugia pahangi and Brugia patei of Brugia spp.; Dipetalonema reconditum of Dipetalonema spp.; Dirofilaria immitis of Dirofilaria spp.; Filaria oculi of Filaria spp.; and Onchocerca cervicalis, Onchocerca gibsoni and Onchocerca gutturosa of Onchocerca spp.
(b) Nematodes of the Setariidae family, for example, Setaria digitata, Setaria equina, Setaria labiatopapillosa and Setaria marshalli of Setaria spp.; and Wuchereria bancrofti of Wuchereria spp.; and
(c) Nematodes of the Filariidae family, for example, Parafilaria multipapillosa of Parafilaria spp.; and Stephanofilaria assamensis, Stephanofilaria dedoesi, Stephanofilaria kaeli, Stephanofilaria okinawaensis and Stephanofilaria stilesi of Stephanofilaria spp.

(11) Spirurida nematodes

(a) Nematodes of the Gnathostomatidae family, for example, Gnathostoma doloresi and Gnathostoma spinigerum of Gnathostoma spp.;
(b) Nematodes of the Habronematidae family, for example, Habronema majus, Habronema microstoma and Habronema muscae of Habronema spp.; and Draschia megastoma of Draschia spp.;
(c) Nematodes of the Physalopteridae family, for example, Physaloptera canis, Physaloptera cesticillata, Physaloptera erdocyona, Physaloptera felidis, Physaloptera gemina, Physaloptera papilloradiata, Physaloptera

praeputialis, Physaloptera pseudopraerutialis, Physaloptera rara, Physaloptera sibirica and Physaloptera vulpineus of Physaloptera spp.;
(d) Nematodes of the Gongylonematidae family, for example, Gongylonema pulchrum of Gongylonema spp.;
(e) Nematodes of the Spirocercidae family, for example, Ascarops strongylina of Ascarops spp.; and
(f) Nematodes of the Thelaziidae family, for example, Thelazia callipaeda, Thelazia gulosa, Thelazia lacrymalis, Thelazia rhodesi and Thelazia skrjabini of Thelazia spp.

[Formulation of Controlling Other Harmful Organisms]

In addition, formulations for controlling harmful organisms of the present invention exhibit excellent control effects on other harmful insects have a sting or venom that can harm humans and animals, harmful insects carrying various pathogens and pathogenic bacteria, and harmful insects that impart discomfort to humans (such as toxic harmful insects, sanitary harmful insects, and unpleasant harmful insects).
Specific examples of these other harmful insects are listed below.

(1) Hymenoptera harmful insects

Sawflies of the Argidae family, wasps of the Cynipidae family, sawflies of the Diprionidae family, ants of the Formicidae family, wasps of the Mutillidae vamily family, and wasps of the Vespidae family.

(2) Other harmful insects

Blattodea, termites, Araneae, centipedes, millipedes, crustacea and Cimex lectularius.

EXAMPLES

[Formulations]

Several examples of formulations for controlling harmful organisms, insecticidal, acaricidal, or nematicidal formulations, endoparasite controlling formulations or parasiticidal formulations of the present invention are described below, but the additives and the addition ratios are not limited to those detailed in these examples, and can be modified over a wide range. The term "parts" in the formulations indicates "parts by weight".
Formulations for agricultural and horticultural use and formulations for paddy rice are described below.

(Formulation 1: Water-Dispersible Powder)

Forty parts of the (hetero)aryl sulfonamide compound of the present invention, 53 parts of diatomaceous earth, 4 parts of a higher alcohol sulfate and 3 parts of an alkylnaphthalene sulfonate salt were mixed together uniformly and then finely pulverized to obtain a water-dispersible powder containing 40% of the active ingredient.

(Formulation 2: Emulsion)

Thirty parts of the (hetero)aryl sulfonamide compound of the present invention, 33 parts of xylene, 30 parts of dimethylformamide and 7 parts of a polyoxyethylene alkyl aryl ether were mixed together and dissolved to obtain an emulsion containing 30% of the active ingredient.

(Formulation 3: Granules)

Five parts of the (hetero)aryl sulfonamide compound of the present invention, 40 parts of talc, 38 parts of clay, 10 parts of bentonite and 7 parts of sodium alkyl sulfate were mixed together uniformly and then finely pulverized, and the resulting powder was granulated into a granular shape having a diameter of 0.5 to 1.0 mm to obtain granules containing 5% of the active ingredient.

(Formulation 4: Granules)

Five parts of the (hetero)aryl sulfonamide compound of the present invention, 73 parts of clay, 20 parts of bentonite, 1 part of sodium dioctylsulfosuccinate and 1 part of potassium phosphate were pulverized and mixed together, water was then added to the resulting powder and thoroughly mixed, and the mixture was granulated and dried to obtain

granules containing 5% of the active ingredient.

(Formulation 5: Suspension)

Ten parts of the (hetero)aryl sulfonamide compound of the present invention, 4 parts of a polyoxyethylene alkyl aryl ether, 2 parts of sodium polycarboxylate, 10 parts of glycerol, 0.2 parts of xanthan gum and 73.8 parts of water were mixed, and the resulting mixture was subjected to wet pulverizing down to a grain size of not more than 3 microns to obtain a suspension containing 10% of the active ingredient.

Formulations for endoparasite control and parasiticidal formulations are described below.

(Formulation 6: Granulated Powder)

Five parts of the (hetero)aryl sulfonamide compound of the present invention was dissolved in an organic solvent to obtain a solution, the solution was sprayed onto 94 parts of kaolin and 1 part of white carbon, and the solvent was then evaporated under reduced pressure. This type of granulated powder can be mixed with animal feed.

(Formulation 7: Injection)

From 0.1 to 1 part of the (hetero)aryl sulfonamide compound of the present invention and 99 to 99.9 parts of peanut oil were mixed together uniformly, and the resulting mixture was then filter-sterilized using a sterilizing filter.

(Formulation 8: Pour-on Formulation)

Five parts of the (hetero)aryl sulfonamide compound of the present invention, 10 parts of a myristate ester and 85 parts of isopropanol were mixed together uniformly to obtain a pour-on formulation.

(Formulation 9: Spot-on Formulation)

From 10 to 15 parts of the (hetero)aryl sulfonamide compound of the present invention, 10 parts of a palmitate ester and 75 to 80 parts of isopropanol were mixed together uniformly to obtain a spot-on formulation.

(Formulation 10: Spray-on Formulation)

One part of the (hetero)aryl sulfonamide compound of the present invention, 10 parts of propylene glycol and 89 parts of isopropanol were mixed together uniformly to obtain a spray-on formulation.

Examples of the compounds are described below in order to describe the present invention in more detail. However, it should be understood that the present invention is not limited to the examples of the compounds described below.

[Example 1]

Synthesis of 1,1,1-trifluoro-N-[5-(trifluoromethyl)-2-[3-(trifluoromethyl)pyrazol-1-yl]-3-pyridyl]methanesulfonamide (Compound No. (1-1))

(Step 1)

3-(Trifluoromethyl)pyrazole → 3-Nitro-5-(trifluoromethyl)-2-[3-(trifluoromethyl)pyrazol-1-yl]pyridine

[Chem. 12]

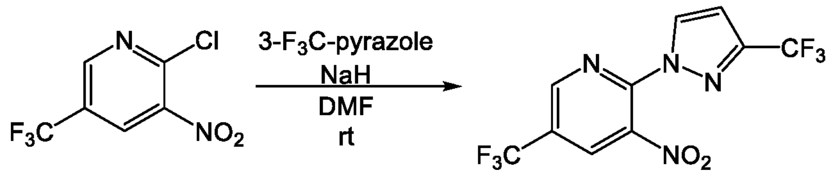


3-(Trifluoromethyl)pyrazole in an amount of 0.3 g was dissolved in 7 ml of DMF, and 0.11 g of 60% sodium hydride-containing paraffin was added thereto at 0°C. The mixture was stirred for 10 minutes at 0°C. Subsequently, 0.5 g of 2-chloro-3-nitro-5-(trifluoromethyl)pyridine was added thereto at 0°C. The reaction mixture was warmed to room temperature, and stirred for 30 minutes. The obtained reaction solution was poured into water, and subjected to extraction with ethyl acetate. The obtained organic layer was dried over anhydrous magnesium sulfate, and subjected to filtration. The filtrate was concentrated under reduced pressure. Thereby, a concentrated product was obtained.

(Step 2)

3-Nitro-5-(trifluoromethyl)-2-[3-(trifluoromethyl)pyrazol-1-yl]pyridine → 5-(Trifluoromethyl)-2-[3-(trifluoromethyl)pyrazol-1-yl]pyridin-3-amine

[Chem. 13]

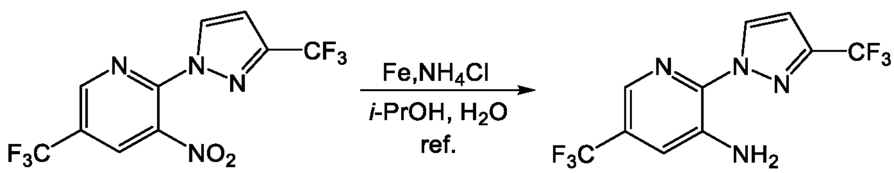


The concentrated product obtained in Step 1 was dissolved in 8.4 ml of isopropyl alcohol, and 2.7 ml of water, 0.54 g of iron powder, and 0.13 g of ammonium chloride were added thereto at room temperature. The reaction mixture was stirred for 40 minutes under reflux conditions. The reaction mixture was cooled to room temperature. Subsequently, ethyl acetate was added thereto. The obtained reaction solution was subjected to filtration through celite. The filtrate was concentrated under reduced pressure to obtain a concentrated product. A saturated aqueous solution of sodium hydrogencarbonate was added to the concentrated product. Subsequently, the mixture was subjected to extraction with ethyl acetate. The obtained organic layer was washed with a saturated aqueous solution of sodium chloride, dried over anhydrous magnesium sulfate, and filtrated. The obtained filtrate was concentrated under reduced pressure, and thereby, a concentrated product was obtained. The obtained concentrated product was purified by column chromatography with silica gel. Thereby, 0.20 g of the target product was obtained (yield: 29%, 2 steps).

The $^1$H-NMR of the obtained target product is shown below.

$^1$H-NMR (400 MHz, $CDCl_3$): δ 8.66 (d, 1H), 8.04 (s, 1H), 7.33 (d, 1H), 6.73 (d, 1H), 5.81 (brs, 2H).

(Step 3)

5-(Trifluoromethyl)-2-[3-(trifluoromethyl)pyrazol-1-yl]pyridin-3-amine → 1,1,1-Trifluoro-N-[5-(trifluoromethyl)-2-[3-(trifluoromethyl)pyrazol-1-yl]-3-pyridyl] methanesulfonamide

[Chem. 14]

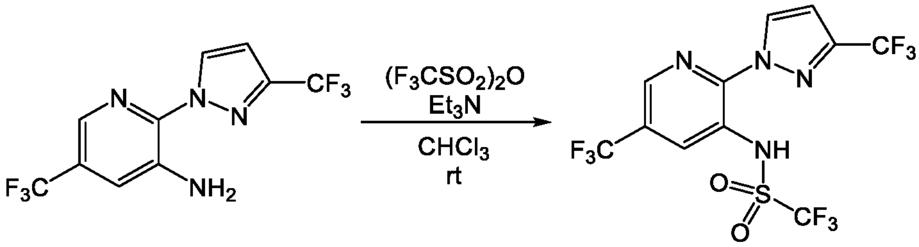


5-Trifluoromethyl-2-[3-(trifluoromethyl)pyrazol-1-yl]pyridin-3-amine in an amount of 0.19 g, and 0.10g of trimethylamine were dissolved in 8 ml of chloroform, and 0.27 g of anhydrous trifluoromethanesulfonic acid was dropwise added thereto at 0°C. The reaction mixture was warmed up to room temperature, and stirred for 5 hours at room temperature. The obtained reaction solution was concentrated under reduced pressure, and water was added to the obtained concentrated product. The organic layer thereof was extracted with ethyl acetate. The obtained organic layer was washed with a saturated aqueous solution of sodium chloride, dried over anhydrous magnesium sulfate, and filtrated. The filtrate was concentrated under reduced pressure. The obtained concentrated product was purified by column chromatography with silica gel. Thereby, 0.17 g of the target product was obtained (yield: 62%).

The $^1$H-NMR of the obtained target product is shown below.

$^1$H-NMR (400 MHz, DMSO-$d_6$): δ 8.45 (dd, 1H), 8.28 (d, 1H), 8.15 (d, 1H), 6.88 (d, 1H).

Examples of the (hetero)aryl sulphonamide compounds of the present invention produced by the same methods as those described in the above Examples are shown in Table 1 to Table 3. Table 2 indicates the substituents of the compounds represented by Formula (I-1), and Table 3 indicates the substituents of the compounds represented by Formula (1-2). The physical data of the compounds are described in the column of "Physical property". In the tables, "Me" indicates a methyl group, "Et" indicates an ethyl group, "cPr" indicates a cyclopropyl group, and "Ph" indicates a phenyl group.

Table 1

| Compound No. | Structure | Physical property |
|---|---|---|
| 1-1 | 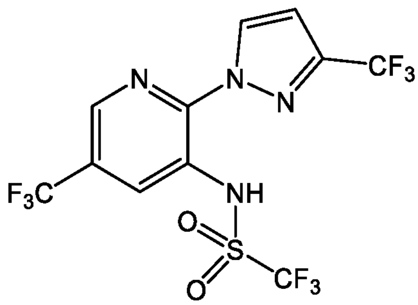 | m.p. 270 - 272°C |
| 1-2 | 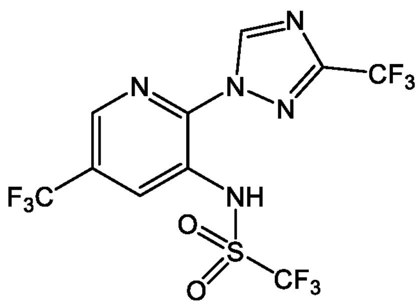 | amorphous |

(continued)

| Compound No. | Structure | Physical property |
|---|---|---|
| 1-3 | 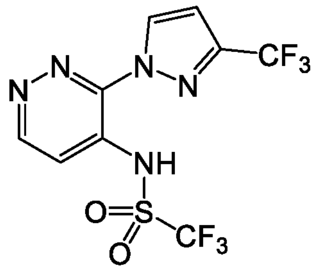 | m.p. 242 - 249°C |
| 1-4 | 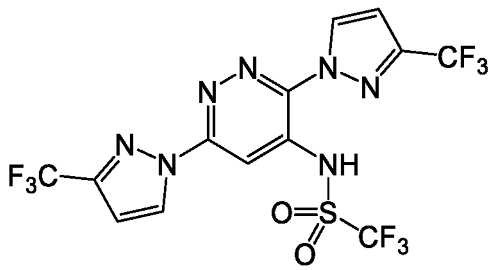 | m.p. 234 - 255°C |
| 1-5 | 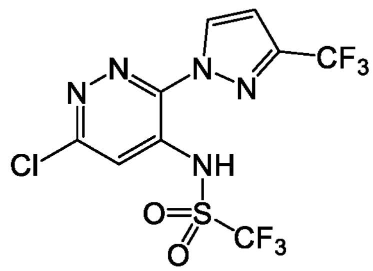 | m.p. 286 - 292°C |
| 1-6 | 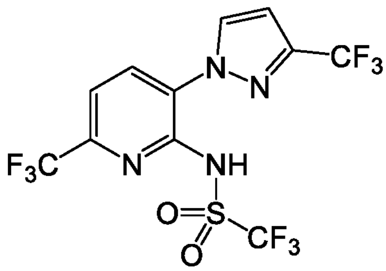 | viscous oil |
| 1-7 | 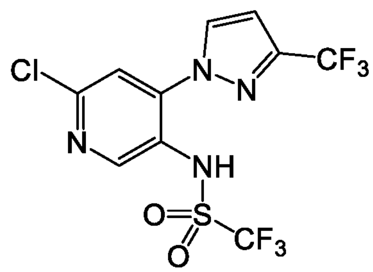 | m.p. 142 - 144°C |

(continued)

| Compound No. | Structure | Physical property |
|---|---|---|
| 1-8 | 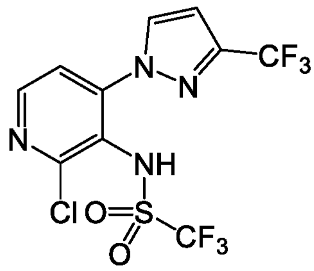 | viscous oil |
| 1-9 | 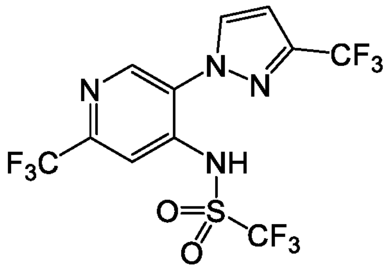 | m.p. 290°C up |
| 1-10 | 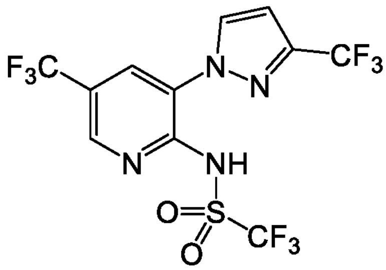 | m.p.114-134°C |
| 1-11 | 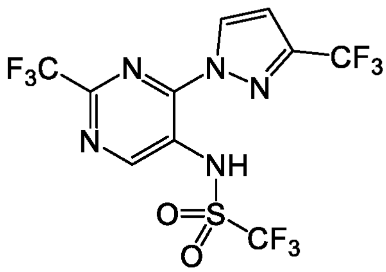 | m.p. 69 - 70°C |
| 1-12 | 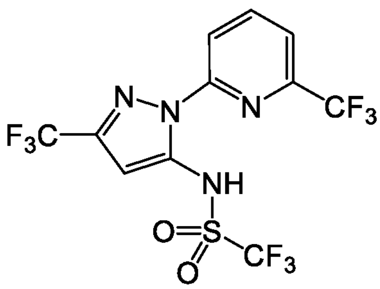 | m.p. 89 - 90°C |

(continued)

| Compound No. | Structure | Physical property |
|---|---|---|
| 1-13 | 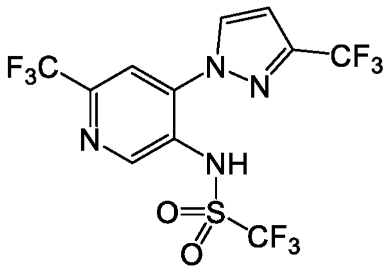 | m.p. 85 - 86°C |
| 1-14 | 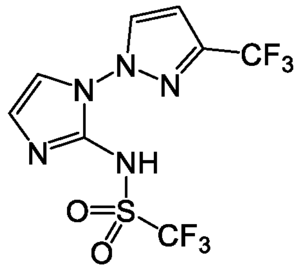 | m.p. 148 - 149°C |
| 1-15 | 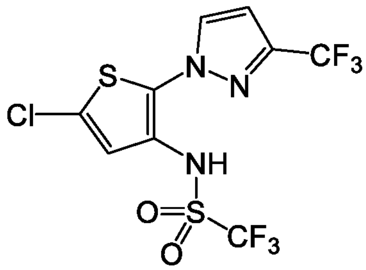 | m.p. 94 - 95°C |

[Chem. 15]

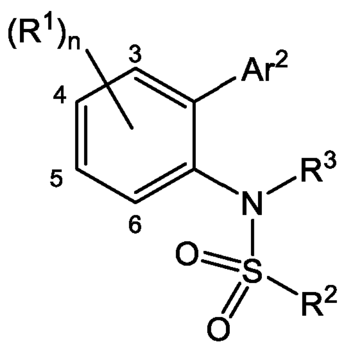

(I-1)

Table 2

| Compound No. | $(R^1)_n$ | R2 | R3 | $Ar^2$ | Physical property |
|---|---|---|---|---|---|
| 2-1 | 4-Br | $CF_3$ | H | 1 *H*-benzo[d]imidazol-2-yl | m.p. 220°C up |
| 2-2 | 4-Br | $CF_3$ | $SO_2CF_3$ | 1 *H*-benzo[d]imidazol-2-yl | m.p. 180 - 182°C |
| 2-3 | 5-$CF_3$ | $CF_3$ | H | 5,6-$Cl_2$-1 *H*-benzo[d]imidazol-2-yl | m.p. 220°C up |
| 2-4 | 5-$CF_3$ | $CF_3$ | H | 5-Cl-7-COOMe-1 *H*-benzo[d] imidazol-2-yl | m.p. 220°C up |

(continued)

| Compound No. | $(R^1)_n$ | R2 | R3 | $Ar^2$ | Physical property |
|---|---|---|---|---|---|
| 2-5 | 5-$CF_3$ | $CF_3$ | $SO_2CF_3$ | 5-$CF_3$-1 *H*-benzo[d]imidazol-2-yl | amorphous |
| 2-6 | 5-$CF_3$ | $CF_3$ | H | 5-$CF_3$-1 *H*-benzo[d]imidazol-2-yl | m.p. 220°C up |
| 2-7 | 5-$CF_3$ | $CF_3$ | H | 4,5-$F_2$-7-COOMe-1 *H*-benzo[d] imidazol-2-yl | m.p. 220°C up |
| 2-8 | 5-$CF_3$ | $CF_3$ | H | 5,6-$F_2$-1 *H*-benzo[d]imidazol-2-yl | m.p. 220°C up |
| 2-9 | 4,5-$F_2$ | $CF_3$ | H | 5,6-$F_2$-1 *H*-benzo[d]imidazol-2-yl | m.p. 220°C up |
| 2-10 | 5-$CF_3$ | $CF_3$ | H | 5-Cl-benzo[d]oxazol-2-yl | m.p. 131 - 133°C |
| 2-11 | 5-$CF_3$ | $CH_2CF_3$ | H | 5,6-$F_2$-1 *H*-benzo[d]imidazol-2-yl | m.p. 220°C up |
| 2-12 | 5-$CF_3$ | $CF_3$ | H | 1*H*-pyrazol-1-yl | m.p. 110 - 112°C |
| 2-13 | 4-$CF_3$ | $CF_3$ | H | 1*H*-pyrazol-1-yl | m.p. 220°C up |
| 2-14 | 5-$CF_3$ | Et | H | 5,6-$F_2$-1 *H*-benzo[d]imidazol-2-yl | m.p. 220°C up |
| 2-15 | 5-$CF_3$ | $CF_3$ | H | 3-(4-Br-Ph)-1*H*-1,2,4-triazol-1-yl | amorphous |
| 2-16 | 5-$CF_3$ | $CF_3$ | H | 4-(4-Cl-Ph)-1 *H*-pyrazol-1-yl | m.p. 140 - 142°C |
| 2-17 | 5-$CF_3$ | Et | H | 3-(4-Br-Ph)-1*H*-1,2,4-triazol-1-yl | m.p. 221 - 223°C |
| 2-18 | 5-$CF_3$ | $CF_3$ | H | 3-(4-Cl-Ph)-1 *H*-pyrazol-1-yl | m.p. 143 - 145°C |
| 2-19 | 5-$CF_3$ | $CF_3$ | H | 4-Br-1 *H*-pyrazol-1-yl | m.p. 103 - 105°C |
| 2-20 | 5-$CF_3$ | $CF_3$ | H | 3-(3,4-$F_2$-Ph)-1 *H*-pyrazol-1-yl | m.p. 157 - 159°C |
| 2-21 | 5-$CF_3$ | $CF_3$ | H | 3-$CF_3$-1*H*-pyrazol-1-yl | amorphous |
| 2-22 | 5-$CF_3$ | $CF_3$ | H | 3-(4-$CF_3$-Ph)-1 *H*-pyrazol-1-yl | m.p. 136 - 138°C |
| 2-23 | 5-$CF_3$ | $CF_3$ | H | 5-$CF_3$-1,3,4-oxadiazol-2-yl | m.p. 270°C up |
| 2-24 | 5-$CF_3$ | $CF_3$ | H | 3-Me-1,2,4-oxadiazol-5-yl | m.p. 90 - 91 °C |
| 2-25 | 5-$CF_3$ | $CF_3$ | H | 3-(4-Cl-Ph)-1,2,4-oxadiazol-5-yl | m.p. 130 - 132°C |
| 2-26 | 4-Br | $CF_3$ | H | 3-$CF_3$-1 *H*-pyrazol-1-yl | m.p. 169 -171°C |
| 2-27 | 5-$CF_3$ | $CF_3$ | H | 5-(4-Cl-Ph)-1,2,4-oxadiazol-3-yl | m.p. 85 - 87°C |
| 2-28 | 5-$CF_3$ | $CF_3$ | H | 5-(thiophen-2-yl)-1,2,4-oxadiazol-3-yl | m.p. 113-115°C |
| 2-29 | 5-$CF_3$ | $CF_3$ | H | 5-$CF_3$-1,2,4-oxadiazol-3-yl | m.p. 183 - 185°C |
| 2-30 | 5-$CF_3$ | $CF_3$ | H | 5-$^c$Pr-1,2,4-oxadiazol-3-yl | m.p. 100 - 102°C |
| 2-31 | 4-Br | Me | H | 3-$CF_3$-1 H-pyrazol-1-yl | viscous oil |
| 2-32 | 4-(2-Cl-4-$CF_3$-Ph) | Me | H | 3-$CF_3$-1 *H*-pyrazol-1-yl | m.p. 121 - 123°C |
| 2-33 | 5-$CF_3$ | $CF_3$ | H | 3-(4-Cl-Ph)-4-Me-1*H*-pyrazol-1-yl | m.p. 121 - 123°C |
| 2-34 | 5-$CF_3$ | $CF_3$ | H | 4-$CF_3$-1 *H*-imidazol-2-yl | m.p. 154 - 156°C |
| 2-35 | 5-$CF_3$ | $CF_3$ | H | 6-$CF_3$-pyridin-2-yl | m.p. 83 - 84°C |
| 2-36 | 5-$CF_3$ | $CF_3$ | H | 5-$CF_3$-pyridin-2-yl | m.p. 121 - 123°C |
| 2-37 | 5-$CF_3$ | $CF_3$ | H | 6-$CF_3$-pyridin-3-yl | m.p. 197 - 199°C |
| 2-38 | 5-$CF_3$ | $CF_3$ | H | 2-$CF_3$-pyrimidin-5-yl | m.p. 105 - 107°C |
| 2-39 | 5-$CF_3$ | $CF_3$ | H | 5-Me-thiazol-2-yl | m.p. 122 - 124°C |
| 2-40 | 5-$CF_3$ | $CF_3$ | H | 4-Me-thiazol-2-yl | m.p. 82 - 83°C |

[Chem. 16]

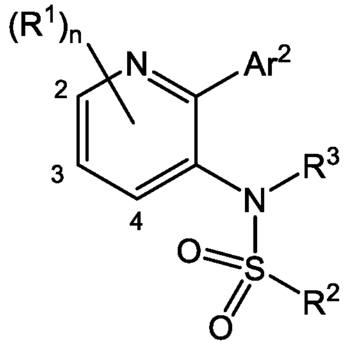

(I-2)

Table 3

| Compound No. | $(R^1)_n$ | R2 | R3 | $Ar^2$ | Physical property |
|---|---|---|---|---|---|
| 3-1 | 3-$CF_3$ | $CF_3$ | H | 3-COOMe-1H-pyrazol-1-yl | m.p. 270°C up |
| 3-2 | 3-$CF_3$ | $CF_3$ | H | 3-C(=O)$NH^iPr$-1 H-pyrazol-1-yl | m.p. 270°C up |
| 3-3 | 3-$CF_3$ | $CF_3$ | H | 3-(4-Cl-phenyl)-1 H-pyrazol-1-yl | m.p. 184 - 186°C |
| 3-4 | 3-$CF_3$ | Me | H | 3-$CF_3$-1 H-pyrazol-1-yl | m.p. 158 - 160°C |
| 3-5 | 3-$CF_3$ | $CF_3$ | H | 3-$CF_3$-1 H-pyrazol-1-yl | m.p. 184 - 186°C |
| 3-6 | 3-Cl | $CF_3$ | H | 3-$CF_3$-1 H-pyrazol-1-yl | m.p. 277 - 281°C |
| 3-7 | 3-$CF_3$ | $CF_3$ | Me | 3-$CF_3$-1 H-pyrazol-1-yl | m.p. 83 - 88°C |
| 3-8 | 3-$CF_3$ | $CH_2CF_3$ | H | 3-$CF_3$-1 H-pyrazol-1-yl | m.p. 81 - 85°C |
| 3-9 | - | $CF_3$ | H | 3-$CF_3$-1 H-pyrazol-1-yl | m.p. 266 - 267°C |
| 3-10 | 3-Me | $CF_3$ | H | 3-$CF_3$-1 H-pyrazol-1-yl | m.p. 260 - 264°C |
| 3-11 | 3-$CF_3$ | $CF_3$ | H | 3-$CF_3$-1 H-pyrazol-1-yl | m.p. 60 - 64°C |
| 3-12 | 2-Cl | $CF_3$ | H | 3-$CF_3$-1 H-pyrazol-1-yl | m.p. 243 - 245°C |
| 3-13 | 3-I | $CF_3$ | H | 3-$CF_3$-1 H-pyrazol-1-yl | m.p.98-101°C |
| 3-14 | 3-CN | $CF_3$ | H | 3-$CF_3$-1H-pyrazol-1-yl | m.p. 285°C up |
| 3-15 | 2-Me | $CF_3$ | H | 3-$CF_3$-1 H-pyrazol-1-yl | m.p. 235 - 243°C |
| 3-16 | 2-(6-$CF_3$-pyridin-3-yl) | $CF_3$ | H | 3-$CF_3$-1H-pyrazol-1-yl | m.p. 285°C up |
| 3-17 | 3-$CF_3$ | $CCl_3$ | H | 3-$CF_3$-1H-pyrazol-1-yl | m.p. 151 -153°C |
| 3-18 | 2-OMe | $CF_3$ | H | 3-$CF_3$-1 H-pyrazol-1-yl | viscous oil |
| 3-19 | 4-Me | $CF_3$ | H | 3-$CF_3$-1 H-pyrazol-1-yl | m.p. 110 - 112°C |
| 3-20 | 2-$CF_3$ | $CF_3$ | H | 3-$CF_3$-1 H-pyrazol-1-yl | amorphous |
| 3-21 | 3-$CF_3$ | $CF_3$ | H | 1 H-pyrazol-1-yl | m.p. 234 - 238°C |
| 3-22 | 3-$CF_3$ | $CF_3$ | H | 3-Cl-1 H-pyrazol-1-yl | m.p. 272 - 274°C |
| 3-23 | 3-$CF_3$ | $CF_3$ | H | 3-$^tBu$-1 H-pyrazol-1-yl | m.p. 94 - 96°C |
| 3-24 | 3-$CF_3$ | $CF_3$ | H | 3-Br-1 H-pyrazol-1-yl | m.p. 89 - 90°C |
| 3-25 | 3-$CF_3$ | $CF_3$ | H | 3-I-1 H-pyrazol-1-yl | m.p. 104 - 106°C |

(continued)

| Compound No. | $(R^1)_n$ | R2 | R3 | $Ar^2$ | Physical property |
|---|---|---|---|---|---|
| 3-26 | 3-$CF_3$ | $CF_3$ | H | 3-$CF_3$-5-$CF_3$-1 H-pyrazol-1-yl | viscous oil |
| 3-27 | 3-$CF_3$ | $CF_3$ | H | 4-F-1H-pyrazol-1-yl | m.p. 103 - 104°C |
| 3-28 | 3-$CF_3$ | $CF_3$ | H | 4-Cl-1 H-pyrazol-1-yl | m.p. 87 - 88°C |
| 3-29 | 3-$CF_3$ | $CF_3$ | H | 4-Br-1 H-pyrazol-1-yl | m.p. 102 - 103°C |
| 3-30 | 3-$CF_3$ | $CF_3$ | H | 4-$CF_3$-1 H-pyrazol-1-yl | m.p. 92 - 94°C |
| 3-31 | 3-$CF_3$ | $CF_3$ | H | 4-CN-1 H-pyrazol-1-yl | m.p. 116 - 118°C |
| 3-32 | 3-$CF_3$ | $CF_3$ | H | 5-Me-1 H-pyrazol-1-yl | $n_D$(22.0°C) 1.4964 |
| 3-33 | 3-$CF_3$ | $CF_3$ | H | 4-$CF_3$-1 H-imidazol-1-yl | viscous oil |
| 3-34 | 2-C(=O)$NH_2$ | $CF_3$ | H | 3-$CF_3$-1 H-pyrazol-1-yl | m.p. 202 - 206°C |
| 3-35 | 2-(2-Cl-4-$CF_3$-phenyl) | $CF_3$ | H | 3-$CF_3$-1 H-pyrazol-1-yl | viscous oil |
| 3-36 | 2-CN | $CF_3$ | H | 3-$CF_3$-1 H-pyrazol-1-yl | m.p. 290°C up |
| 3-37 | 3-$CF_3$ | $CH_2CF_2CH_3$ | H | 3-$CF_3$-1 H-pyrazol-1-yl | m.p. 104 - 105°C |
| 3-38 | 3-$CF_3$ | $CF_3$ | H | 3-$CF_3$-4-Br-1 H-pyrazol-1-yl | m.p. 106 - 108°C |
| 3-39 | 3-$CF_3$ | $CF_3$ | H | 3-$CF_3$-5-Me-1 H-pyrazol-1-yl | $n_D$(21.6°C) 1.4534 |
| 3-40 | 2-SMe | $CF_3$ | H | 3-$CF_3$-1 H-pyrazol-1-yl | $n_D$(18.4°C) 1.5191 |
| 3-41 | 3-$CF_3$ | $CF_3$ | H | 1-$CH_2CF_3$-1 H-pyrazol-4-yl | viscous oil |
| 3-42 | 3-$CF_3$ | $CF_3$ | $SO_2CF_3$ | 1-$CH_2CF_3$-1 H-pyrazol-4-yl | m.p. 119 - 120°C |
| 3-43 | 3-$CF_3$ | $CF_3$ | H | 3-$CF_3$-5-$^t$Bu-1 H-pyrazol-1-yl | m.p. 73 - 74°C |
| 3-44 | 3-$CF_3$ | $CF_3$ | H | 3-CN-1 H-pyrazol-1-yl | m.p. 83 - 84°C |
| 3-45 | 3-$CF_3$ | $CF_3$ | H | 3-$SOCF_3$-1H-pyrazol-1-yl | m.p. 220°C up |
| 3-46 | 3-$CF_3$ | $CF_3$ | H | 3-$CF_3$-4-Br-5-Me-1 H-pyrazol-1-yl | $n_D$(21.0°C) 1.4952 |
| 3-47 | 3-$CF_3$ | $CF_3$ | H | 6-$CF_3$-pyridin-2-yl | m.p. 92 - 93°C |
| 3-48 | 3-$CF_3$ | $CF_3$ | H | 1-$CH_2CF_3$-1H-pyrazol-3-yl | m.p. 135-136°C |
| 3-49 | 3-$CF_3$ | $CF_3$ | H | 3-$CF_3$-5-$NH_2$-1 H-pyrazol-1-yl | m.p. 81 - 83°C |

Among the compounds shown in Table 1, Table 2, and Table 3, the $^1$H-NMR data of the compounds having a viscous oil property or amorphous property are shown below.

Compound No. (1-2): $^1$H-NMR (400 MHz, DMSO-$d_6$): δ 9.29 (s, 1H), 8.34 (d, 1H), 8.17 (d, 1H).

Compound No. (2-5): 1H-NMR (400 MHz, DMSO-$d_6$): δ 8.33 (s, 1H), 8.02 (m, 1H), 7.93 (m, 1H), 7.79 (s, 1H), 7.47 (d, 1H), 7.12 (d, 1H).

Compound No. (2-15): $^1$H-NMR (400 MHz, DMSO-$d_6$): δ 9.32 (s, 1H), 8.00 (d, 2H), 7.84 (m, 2H), 7.67 (d, 2H), 7.23 (d, 1H).

Compound No. (2-21): $^1$H-NMR (400 MHz, DMSO-$d_6$): δ 8.59 (s, 1H), 7.82 (s, 1H), 7.66 (d, 1H), 7.19 (d, 1H), 6.88 (s, 1H).

Compound No. (2-31): $^1$H-NMR (400 MHz, $CDCl_3$): δ 8.58 (s, 1H), 7.89 (s, 1H), 7.67 (d, 1H), 7.56 (m, 2H), 6.81 (d, 1H).

Compound No. (1-6): $^1$H-NMR (400 MHz, MeOH-$d_4$): δ 8.92 (s, 1H), 8.17 (d, 1H), 7.34 (d, 1H), 6.79 (s, 1H).

Compound No. (1-8): $^1$H-NMR (400 MHz, MeOH-$d_4$): δ 8.61 (d, 1H), 8.07 (d, 1H), 7.63 (d, 1H), 6.79 (d, 1H).
Compound No. (3-18): $^1$H-NMR (400 MHz, MeOH-$d_4$): δ 8.61 (s, 1H), 7.92 (d, 1H), 6.92 - 6.89 (m, 2H), 4.01 (s, 3H).
Compound No. (3-20): $^1$H-NMR (400 MHz, MeOH-$d_4$): δ 8.48 (d, 1H), 8.24 (d, 1H), 7.74 (d, 1H), 6.81(d, 1H).
Compound No. (3-26): $^1$H-NMR (400 MHz, MeOH-$d_4$): δ 8.53 (d, 1H), 8.44 (d, 1H), 7.40 (s, 1H).
Compound No. (3-33): $^1$H-NMR (400 MHz, MeOH-$d_4$): δ 8.91 (s, 1H), 8.53 (dd, 1H), 8.29 - 8.26 (m, 2H).
Compound No. (3-35): $^1$H-NMR (400 MHz, MeOH-$d_4$): δ 8.71 (dd, 1H), 8.26 (d, 1H), 7.93-7.89 (m, 3H), 7.77 (m, 1H), 6.94 (d, 1H).
Compound No. (3-41): $^1$H-NMR (400 MHz, $CDCl_3$): δ 9.02 (s, 1H), 8.24 (s, 1H), 8.23 (s, 1H), 7.90 (s, 1H), 4.82 (q, 2H).

[Biological Testing]

The test examples described below demonstrate that the (hetero)aryl sulfonamide compounds of the present invention are useful as the active ingredient in a formulation for controlling harmful organisms. The unit "parts" is based on weight.

(Preparation of Test Emulsion)

Five parts of the (hetero)aryl sulfonamide compound of the present invention, 93.6 parts of dimethylformamide and 1.4 parts of a polyoxyethylene alkyl aryl ether were mixed together and dissolved to obtain an emulsion (I) containing 5% of the active ingredient.
For the control, 93.6 parts of dimethylformamide and 1.4 parts of a polyoxyethylene alkyl aryl ether were mixed together and dissolved to obtain an emulsion (II).
The insect mortality rate and the nematode mortality rate were calculated using the following equations.

$$\text{Insect mortality rate (\%)} = (\text{number of dead insects / number of test insects}) \times 100$$

$$\text{Nematode mortality rate (\%)} = (\text{number of dead nematodes / number of test nematodes}) \times 100$$

(Test Example 1)

Efficacy Test against *Mythimna separata*

First, 0.8 g of a commercially-available artificial feed (Insecta LFS, manufactured by Nosan Corporation) and 1 μl of the emulsion (I) were mixed well, and thereby, a test feed was obtained. Subsequently, 0.2 g of the test feed was placed in each of the treatment areas of a plastic test container (volume: 1.4 ml). Subsequently, two second-instar larvae of *Mythimna separata* were inoculated into each treatment area, and a plastic lid was placed on the test container to prevent the larvae from escaping. The closed container was placed in a thermostatic chamber at 25°C. On the 5$^{th}$ day therefrom, the insect mortality rate and the amount of feed consumed were checked. The test was repeated.
In addition, for a control area, the insect mortality rate and the amount of feed consumed were checked in the same manner as that described in Test Example 1, with the exception of replacing the emulsion (I) with the emulsion (II).
Efficacy tests against *Mythimna separata* were conducted for the compounds of Compound Nos. 1-13, 2-3, 2-5, 2-6, 2-8, 2-9, 2-14, 2-15, 2-16, 2-17, 2-18, 2-22, 2-25, 2-36, 3-8, 3-46, and 3-47. For all of the compounds, the insect mortality rate against *Mythimna separata* was 100%, and the amount of feed consumed was 10% or less of the amount of feed consumed in the control area. It can be seen that the (hetero)aryl sulfonamide compounds of the present invention are effective against *Mythimna separata.*

(Test Example 2)

Efficacy Test against *Spodoptera litura*

The emulsion (I) was diluted with water to achieve a concentration of the compound of the present invention

of 125 ppm. Cabbage leaves were soaked in the diluted liquid for 30 seconds. These cabbage leaves were then placed in a Petri dish, and five second-instar larvae of *Spodoptera litura* were released into the Petri dish. The Petri dish was placed in a thermostatic chamber at a temperature of 25°C and a humidity of 60%. Mortality was investigated 6 days after larvae release, and the mortality rate was calculated. The test was repeated.

Efficacy tests against *Spodoptera litura* were conducted for the compounds of Compound Nos. 2-5, 2-6, 2-8, 2-17, and 2-18. All of the compounds exhibited a mortality rate against *Spodoptera litura* of 80% or more.

(Test Example 3)

Efficacy Test against *Plutella xylostella*

The emulsion (I) was diluted with water to achieve a concentration of the compound of the present invention of 125 ppm. Cabbage leaves were soaked in the diluted liquid for 30 seconds. These cabbage leaves were then placed in a Petri dish, and five second-instar larvae of *Plutella xylostella* were released into the Petri dish. The Petri dish was placed in a thermostatic chamber at a temperature of 25°C and a humidity of 60%. Mortality was investigated 3 days after larvae release, and the mortality rate was calculated. The test was repeated.

Efficacy tests against *Plutella xylostella* were conducted for the compounds of Compound Nos. 2-5, 2-6, 2-8, 2-14, 2-17, and 2-18. All of the compounds exhibited a mortality rate against *Plutella xylostella* of 80% or more.

(Test Example 4)

Efficacy Test against *Caenorhabditis elegans* (*In Vitro* Test)

A suspension containing about 50 mixed instars of *Caenorhabditis elegans* per 0.2 mL was dispensed into each well of a 96-well microplate, at a rate of 200 μL of the suspension per well. Subsequently, a solution of the compound of the present invention dissolved in DMSO at a concentration of 10,000 pm was added thereto in an amount of 1.0 μL of the DMSO solution of the compound mentioned above per well. The 96-well microplate was allowed to stand for 2 days at 25°C. Subsequently, mortality was investigated, and the nematode mortality rate was calculated. Observation was performed for 10 seconds, and those individuals that showed no movement during the 10-second observation were deemed to be dead. The test was repeated.

Efficacy tests against *Caenorhabditis elegans* were conducted for the compounds of Compound Nos. 2-3, 2-4, 2-6, 2-7, 2-8, 2-9, 2-10, 2-15, 2-16, and 2-29. All of the compounds exhibited a nematode mortality rate against *Caenorhabditis elegans* of 80% and more.

(Test Example 5)

Efficacy Test against *Meloidogyne incognita* (*In Vitro* Test)

A suspension containing about 50 second-instar larvae (L2) of *Meloidogyne incognita* per 0.2 mL was dispensed into each well of a 96-well microplate, at a rate of 200 μL of the suspension per well. Subsequently, a solution of the compound of the present invention dissolved in DMSO at a concentration of 10,000 pm was added thereto in an amount of 1.0 μL of the DMSO solution of the compound mentioned above per well. The 96-well microplate was allowed to stand for 2 days at 15°C. Subsequently, mortality was investigated, and the nematode mortality rate was calculated. Observation was performed for 10 seconds, and those individuals that showed no movement during the 10-second observation were deemed to be dead. The test was repeated.

Efficacy tests against *Meloidogyne incognita* were conducted for the compounds of Compound Nos. 1-1, 1-4, 1-11, 1-13, 2-3, 2-8, 2-9, 2-11, 2-15, 2-16, 2-18, 2-19, 2-20, 2-21, 2-22, 2-25, 2-26, 2-34, 2-35, 2-36, 3-3, 3-5, 3-6, 3-8, 3-12, 3-13, 3-20, 3-22, 3-24, 3-25, 3-27, 3-28, 3-29, 3-30, 3-31, 3-33, 3-34, 3-35, 3-37, 3-38, 3-39, 3-43, 3-46, 3-47, and 3-48. All of the compounds exhibited a nematode mortality rate against *Meloidogyne incognita* of 80% or more.

(Test Example 6)

Efficacy Test against *Heterodera glycines* (*In Vitro* Test)

A suspension containing about 50 second-instar larvae (L2) of *Heterodera glycines* per 0.2 mL was dispensed into each well of a 96-well microplate, at a rate of 200 μL of the suspension per well. Subsequently, a solution of the compound of the present invention dissolved in DMSO at a concentration of 10,000 pm was added thereto in an amount of 1.0 μL of the DMSO solution of the compound mentioned above per well. The 96-well microplate was allowed to stand

for 3 days at 25°C. Subsequently, mortality was investigated, and the nematode mortality rate was calculated. Observation was performed for 10 seconds, and those individuals that showed no movement during the 10-second observation were deemed to be dead. The test was repeated.

Efficacy tests against *Heterodera glycines* were conducted for the compounds of Compound Nos. 1-1, 2-3, 2-19, 2-20, 2-21, 2-25, 2-26, 2-34, 3-8, and 3-38. All of the compounds exhibited a nematode mortality rate against *Heterodera glycines* of 80% or more.

(Test Example 7)

Efficacy Test against *Meloidogyne incognita* (Club Root Formation Suppression Test)

The emulsion (I) was diluted with water to achieve a concentration of the compound of the present invention of 125 ppm. A plastic container with a diameter of 5 cm was filled with 7 g of medium, and cucumber seeds were planted therein. The culture medium was irrigated with 1 ml of the above dilution, and then inoculated with 200 eggs of *Meloidogyne incognita.* The container was then placed in a thermostatic chamber at a temperature of 25°C and a humidity of 60%, and after 14 days, the roots of the cucumber plants were observed, and the number of club roots formed thereon was counted (treated area).

In the same manner as that described above with the exception that the culture medium being irrigated with the above dilute liquid was not carried out, the number of club roots formed thereon was counted (untreated area).

The club root formation suppression ratio (%) was calculated from the number of club roots formed on the roots of the cucumber plants. The test was repeated.

$$\text{Club root formation suppression ratio (\%)} = (1 - Nt/Nc) \times 100$$

Nt: Total number of club roots formed in treated samples after 14 days after repeating twice
Nc: Total number of club roots formed in untreated samples after 14 days after repeating twice

Efficacy tests against *Meloidogyne incognita* were conducted for the compounds of Compound Nos. 1-1, 2-3, 2-8, 2-9, 2-15, 2-16, 2-18, 2-19, 2-20, 2-21, 2-25, 2-26, 2-34, 2-35, 3-3, 3-5, 3-6, 3-8, 3-12, 3-13, 3-20, 3-22, 3-24, 3-25, 3-27, 3-28, 3-29, 3-30, 3-35, 3-37, 3-38, 3-46, and 3-48. All of the compounds exhibited a club root formation suppression ratio against *Meloidogyne incognita* of 80% or more.

Based on the fact that the (hetero)aryl sulfonamide compounds selected randomly from the compounds of the present invention all exhibited the types of effects described above, it can be seen that the condensed heterocyclic compounds of the present invention, including those compounds not exemplified above, have the effects of controlling harmful organisms, and in particular, insecticidal and acaricidal effects or the like. In addition, it can be seen that the compounds of the present invention have the effects of controlling parasites harmful to animals and humans, such as ectoparasites.

INDUSTRIAL APPLICABILITY

The (hetero)aryl sulfonamide compounds according to the present invention have control activity on harmful organisms, and in particular, have excellent insecticidal, acaricidal and/or nematicidal activity, exhibit excellent safety, and can be advantageously synthesized industrially.

The formulations for controlling harmful organisms of the present invention can control harmful organisms which are problematic in view of farm products or for hygiene reasons. The formulations for controlling harmful organisms of the present invention exhibit excellent control effects on agriculturally harmful organisms even with a reduced concentration.

**Claims**

1. A compound represented by formula (I) or a salt thereof:

[Chem. 1]

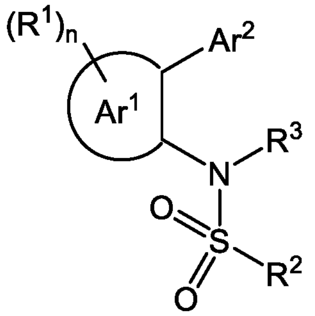

(I)

in formula (I),

$Ar^1$ is a benzene ring or a 5- to 6-membered heteroaryl ring,
$R^1$ is a substituted or unsubstituted C1 to C6 alkyl group, a substituted or unsubstituted C2 to C6 alkenyl group, a substituted or unsubstituted C2 to C6 alkynyl group, a hydroxyl group, a substituted or unsubstituted C1 to C6 alkoxy group, a formyl group, a substituted or unsubstituted C1 to C6 alkylcarbonyl group, a carboxyl group, a substituted or unsubstituted C1 to C6 alkoxycarbonyl group, a substituted or unsubstituted C1 to C6 alkylcarbonyloxy group, a mercapto group, a substituted or unsubstituted C1 to C6 alkylthio group, a substituted or unsubstituted C1 to C6 alkylsulfinyl group, a substituted or unsubstituted C1 to C6 alkylsulfonyl group, a substituted or unsubstituted C3 to C8 cycloalkyl group, a substituted or unsubstituted C6 to C10 aryl group, a substituted or unsubstituted heteroaryl group, a substituted or unsubstituted C6 to C10 aryloxy group, a substituted or unsubstituted heteroaryloxy group, a halogeno group, a nitro group, a cyano group, a group represented by $-NR^aR^b$, a group represented by $-(C{=}O)-NR^cR^d$, or a group represented by $-O-(C{=}O)-NR^cR^d$, n represents the number of $R^1$ and is 0, 1, 2, or 3, and in the case of n being 2 or more, two or more $R^1$ may be the same or different from one another,
each of $R^a$ and $R^b$ independently represents a hydrogen atom, a substituted or unsubstituted C1 to C6 alkyl group, a substituted or unsubstituted C1 to C6 alkylcarbonyl group, or a substituted or unsubstituted C1 to C6 alkoxycarbonyl group,
each of $R^c$ and $R^d$ independently represents a hydrogen atom, or a substituted or unsubstituted C1 to C6 alkyl group,
$R^2$ is a C1 to C6 alkyl group, a C1 to C6 haloalkyl group, a C3 to C8 cycloalkyl group, a C3 to C8 halocycloalkyl group, or a C3 to C8 cycloalkyl C1 to C6 alkyl group, or a C3 to C8 halocycloalkyl C1 to C6 alkyl group,
$R^3$ is a hydrogen atom, a substituted or unsubstituted C1 to C6 alkyl group, a substituted or unsubstituted C1 to C6 alkyl carbonyl group, a substituted or unsubstituted C1 to C6 alkoxy carbonyl group, a substituted or unsubstituted C1 to C6 alkylsulfonyl group, a substituted or unsubstituted C3 to C8 cycloalkyl group, a substituted or unsubstituted C3 to C8 cycloalkyl carbonyl group, or a substituted or unsubstituted C3 to C8 cycloalkoxy carbonyl group, and
$Ar^2$ represents a substituted or unsubstituted heteroaryl group.

**2.** The compound according to Claim 1 or a salt thereof, wherein Formula (I) is Formula (II):

[Chem. 2]

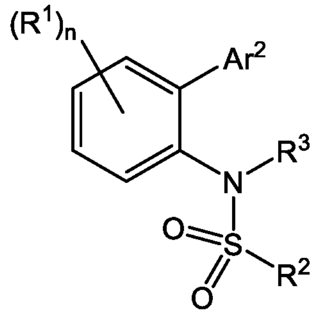

(II)

in Formula (II), $R^1$, $R^2$, $R^3$, $Ar^2$, and n represent the same meanings as those recited in Formula (I).

3. The compound according to Claim 1 or a salt thereof, wherein Formula (I) is Formula (III):

[Chem. 3]

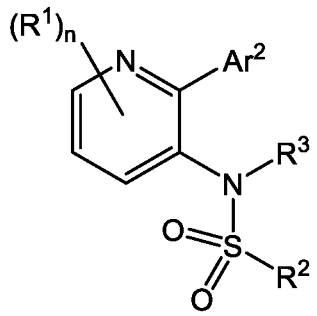

(III)

in Formula (III), $R^1$, $R^2$, $R^3$, $Ar^2$, and n represent the same meanings as those recited in Formula (I).

4. A formulation for controlling harmful organisms, comprising at least one compound selected from the group consisting of the compounds as recited in any one of Claims 1 to 3 and salts thereof, as an active ingredient.

5. An insecticidal or acaricidal formulation, comprising at least one compound selected from the group consisting of the compounds as recited in any one of Claims 1 to 3 and salts thereof, as an active ingredient.

6. A nematicidal formulation, comprising at least one compound selected from the group consisting of the compounds as recited in any one of Claims 1 to 3 and salts thereof, as an active ingredient.

7. A formulation for controlling or exterminating endoparasites, comprising at least one compound selected from the group consisting of the compounds as recited in any one of Claims 1 to 3 and salts thereof, as an active ingredient.

## INTERNATIONAL SEARCH REPORT

International application No.
PCT/JP2019/014789

A. CLASSIFICATION OF SUBJECT MATTER
Int.Cl. [see extra sheet]

According to International Patent Classification (IPC) or to both national classification and IPC

B. FIELDS SEARCHED

Minimum documentation searched (classification system followed by classification symbols)
Int.Cl. C07D231/12, A01N41/06, A01N43/56, A01N47/04, A01P5/00, A01P7/04, A61K31/415, A61K31/4184, A61K31/423, A61K31/4245, A61K31/4439, A61P33/00, C07D235/18, C07D249/08, C07D263/57, C07D271/06, C07D271/10, C07D401/04, C07D403/04, C07D413/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2008-514610 A (SCHERING-PLOUGH LIMITED) 08 May 2008, claims, paragraphs [0472]-[0475] & JP 2010-209083 A & US 2006/0063841 A1 & US 2008/0262048 A1 & WO 2006/034333 A2, tables 9, 9A, claims & CA 2580843 A1 & KR 10-2007-0054691 A & CN 101065352 A | 1, 2, 4-7<br>3 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04.06.2019 | 11.06.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/014789

C (Continuation). DOCUMENTS CONSIDERED TO BE RELEVANT

| Category* | Citation of document, with indication, where appropriate, of the relevant passages | Relevant to claim No. |
|---|---|---|
| X | JP 2007-529439 A (BAYER CROPSCIENCE S.A.) 25 October 2007, claims, table 12, compounds 12-22 to 12-27 & US 2008/0064698 A1 & WO 2005/090314 A1, table 12, compounds 12-22 to 12-27, claims & EP 1727805 A1 & KR 10-2007-0007800 A & CN 1930130 A & KR 10-1179937 B1 | 1-7 |
| X<br>A | JP 2010-508238 A (SCHERING-PLOUGH LIMITED) 18 March 2010, claims, table 20, compounds 93-95, 108 & US 2007/0238700 A1, table 20, compounds 93-95, 108 & WO 2007/116314 A1 & EP 2004595 A1 & CA 2648978 A1 | 1, 2, 4-7<br>3 |
| X<br>A | JP 2010-202648 A (SAGAMI CHEMICAL RESEARCH INSTITUTE) 16 September 2010, table-5, compounds 222, 223 (Family: none) | 1, 2<br>3-7 |
| X<br>A | JP 2014-527973 A (BAYER INTELLECTUAL PROPERTY GMBH) 23 October 2014, table 1, examples 1-456 to 1-490, 1-631 to 1-665, 1-1296 to 1-1330 & US 2014/0329684 A1, table 1, examples 1-456 to 1-490, 1-631 to 1-665, 1-1296 to 1-1330 & WO 2013/041602 A1 & EP 2757886 A1 & CN 103929964 A | 1, 2<br>3-7 |
| X<br>A | THIRUNAVUKKARASU, V. S. et al., Organic Letters, 2013, vol. 15, no. 13, pp. 3286-3289, scheme 1 | 1, 2<br>3-7 |
| X<br>A | DE 4305279 A1 (MERCK PATENT GMBH) 25 August 1994, example 1 (Family: none) | 1, 2<br>3-7 |
| X<br>A | JP 6-345743 A (SANKYO CO.) 20 December 1994, table 1, no. 1, 2 (Family: none) | 1, 2<br>3-7 |
| X<br>A | US 5298189 A (NANOPTICS INCORPORATED) 29 March 1994, claims, claim 6 (Family: none) | 1, 2<br>3-7 |
| X<br>A | JP 2010-518136 A (BASF SE) 27 May 2010, table 5, examples 18, 19 & US 2010/0044688 A1, table 5 & WO 2008/098851 A1 & EP 2139907 A1 & KR 10-2009-0109591 A & CN 101652375 A | 1, 2<br>3-7 |

Form PCT/ISA/210 (continuation of second sheet) (January 2015)

**INTERNATIONAL SEARCH REPORT**

International application No. PCT/JP2019/014789

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2002-161084 A (SUMITOMO PHARMACEUT CO., LTD.) 04 June 2002, examples 143, 144, 146, 147, 149, 150<br>(Family: none) | 1, 2<br>3-7 |
| X<br>A | JP 2015-183129 A (TOYO INK SC HOLDINGS CO., LTD.) 22 October 2015, paragraphs [0051]-[0076]<br>(Family: none) | 1, 2<br>3-7 |
| X<br>A | US 4301169 A (EISAI CO., LTD.) 17 November 1981, example 7<br>& JP 55-66562 A & US 4402966 A & US 4533669 A & US 4602031 A & GB 2044754 A & DE 2946020 A1 & FR 2441616 A1 & CH 642635 A5 | 1, 2<br>3-7 |
| X<br>A | JP 3-89343 A (KONICA CORPORATION) 15 April 1991, page 18, upper left column, compound (9)<br>(Family: none) | 1, 2<br>3-7 |
| X<br>A | JP 2011-518841 A (NEWLINK GENETICS) 30 June 2011, table 1, structure 101<br>& JP 2014-62123 A & JP 2015-187181 A & US 2011/0136796 A1, table 1, structure 101 & US 2014/0323740 A1 & WO 2009/132238 A2 & EP 2291187 A1 & CA 2722159 A1 & CN 102083429 A | 1, 2<br>3-7 |
| X<br>A | WO 2015/173321 A1 (L'OREAL) 19 November 2015, claims, claim 9<br>& FR 3020944 A1 | 1, 2<br>3-7 |
| X<br>A | ZHAO, H. et al., Organic Letters, 2013, vol. 15, no. 19, pp. 5106-5109, scheme 2 | 1, 2<br>3-7 |
| X<br>A | JP 2005-504028 A (AMGEN INC.) 10 February 2005, paragraphs [0153], [0213]<br>& US 2004/0006067 A1, scheme 6, paragraphs [0528], [0529] & WO 2003/009847 A1 & EP 1416933 A1 & CA 2452328 A1 | 1, 2<br>3-7 |
| X<br>A | JP 61-282376 A (BAYER AG) 12 December 1986, page 19, lower left column, reaction formulas<br>& US 4772312 A, column 21, reaction formulas & EP 207285 A1 & DE 3520330 A1 | 1<br>2-7 |
| X<br>A | JP 2-142785 A (MITSUBISHI CHEMICAL CORPORATION) 31 May 1990, example 1<br>(Family: none) | 1<br>2-7 |

Form PCT/ISA/210 (continuation of second sheet) (January 2015)

**INTERNATIONAL SEARCH REPORT**

International application No.
PCT/JP2019/014789

C (Continuation). DOCUMENTS CONSIDERED TO BE RELEVANT

| Category* | Citation of document, with indication, where appropriate, of the relevant passages | Relevant to claim No. |
|---|---|---|
| X<br>A | REGISTRY [online], 21 December 2011, [retrieved on 30 May 2019], retrieved from: STN, CAS registration no. 1351507-41-9 | 1<br>2-7 |
| X<br>A | REGISTRY [online], 21 December 2011, [retrieved on 30 May 2019], retrieved from: STN, CAS registration no. 1351473-81-8 | 1<br>2-7 |
| X<br>A | JP 2011-522806 A (AMGEN INC.) 04 August 2011, paragraph [0477]<br>& US 2011/0092504 A1, example 117 & WO 2009/155121 A2 & EP 2307400 A1 & CA 2725014 A1 | 1, 3<br>2, 4-7 |
| X<br>A | YOSHIZUMI, T. et al., Bioorganic & Medicinal Chemistry Letters, 2008, vol. 18, pp. 3778-3782, table 3, compound 37 | 1, 3<br>2, 4-7 |
| X<br>A | WO 2002/57237 A1 (SHIONOGI AND CO.) 25 July 2002, table 10, no. 213<br>& US 2004/0087604 A1, table 10, no. 213 & EP 1354877 A1 | 1, 2<br>3-7 |

Form PCT/ISA/210 (continuation of second sheet) (January 2015)

**INTERNATIONAL SEARCH REPORT**

International application No.
PCT/JP2019/014789

**Box No. II Observations where certain claims were found unsearchable (Continuation of item 2 of first sheet)**

This international search report has not been established in respect of certain claims under Article 17 (2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
[see extra sheet]

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT

International application No.
PCT/JP2019/014789

(Continuation of Box No. III)

Claim 1 is "a compound represented by formula (I) or a salt thereof", claim 2 which is a dependent claim of claim 1 is "the compound or a salt thereof, according to claim 1, wherein formula (I) is formula (II)", claim 3 which is a dependent claim of claim 1 is "the compound or a salt thereof, according to claim 1, wherein formula (I) is formula (III)", and claims 4-7 are dependent claims of claims 1-3.

The invention in claims 1, 2, and 4-7 lacks novelty in light of document 1 (WO 2006/034333 A1) and document 2 (WO 2005/090314 A1) described as prior art documents in the specification of the present application. Thus, it is obvious that the invention in claims 1 and 2 does not have a special technical feature.

Claims are therefore classified into at least the following four inventions.

(Invention 1) Portion relating to compound of "formula (III)" in claims 1 and 3-7

The structure of the "formula (III)" is not described in documents 1 and 2 and is thus conceivable to be a special technical feature.

Thus, a portion relating to the compound of the "formula (III)" in claims 1 and 3-7 is classified as invention 1.

(Invention 2) Portion relating to compound of "formula (II) where n is 0" in claims 1, 2, and 4-7

A technical feature of the invention relating to the compound of the "formula (II) where n is 0" in common with the invention classified as invention 1 (a portion relating to the compound of the "formula (III)") is a structure of the "formula (I)", and the structure is not a special technical feature as mentioned above. Moreover, there is no same or corresponding special technical feature between them.

In addition, the invention of the portion relating to the compound of the "formula (II) where n is 0" is not substantially identical to or similarly closely related to any of the claims classified as invention 1 and thus cannot be classified as invention 1.

Thus, a portion relating to the compound of the "formula (II) where n is 0" in claims 1, 2, and 4-7 is classified as invention 2.

(Invention 3) Portion relating to compound of "formula (II) where n is 1" in claims 1, 2, and 4-7

A technical feature of the invention of a portion relating to the compound of the "formula (II) where n is 1" in common with the invention classified as invention 1 (a portion relating to the compound of the "formula (III)") is a structure of the "formula (I)". A technical feature of the invention of a portion relating to the compound of the "formula (II) where n is 1" in common with the invention classified as invention 2 (a portion relating to the compound of the "formula (II) where n is 0") is a structure of the "formula (II)". These structures are not special technical features as mentioned above. Moreover, there is no same or corresponding special technical feature between them.

In addition, the portion relating to the compound of the "formula (II) where n is 1" is not substantially identical to or similarly closely related to any of the claims classified as invention 1 or 2 and thus cannot be classified as invention 1 or 2.

Thus, a portion relating to the compound of the "formula (II) where n is 0" in claims 1, 2, and 4-7 is classified as invention 3.

Form PCT/ISA/210 (extra sheet) (January 2015)

**INTERNATIONAL SEARCH REPORT**

International application No.
PCT/JP2019/014789

(Continuation of Box No. III)

(Invention 4) Portion relating to compound of formula I which is neither the "formula (II)" nor the "formula (III)" in claims 1 and 4-7

A technical feature of the invention of a portion relating to the compound of the "formula (I)" which is neither the "formula (II)" nor the "formula (III)" in common with the inventions classified as inventions 1-3 is the structure of the "formula (I)", and the structure is not a special technical feature as mentioned above. Moreover, there is no same or corresponding special technical feature between them.

In addition, the invention of the portion relating to the compound of the "formula (I)" which is neither the "formula (II)" nor the "formula (III)" is not substantially identical to or similarly closely related to any of the claims classified as inventions 1-3 and thus cannot be classified as inventions 1-3.

Thus, the portion relating to the compound of the "formula (I)" which is neither the "formula (II)" nor the "formula (III)" in claims 1 and 4-7 is classified as invention 4.

In addition, please note the followings. In view of the fact that claims 1-7 lack novelty in light of many other documents described in the column C besides documents 1 and 2, claims may be classified as 4 or more inventions.

Form PCT/ISA/210 (extra sheet) (January 2015)

INTERNATIONAL SEARCH REPORT

International application No. PCT/JP2019/014789

<Subject to be searched>

The invention in claim 1 is an invention relating to "a compound represented by formula (I) or a salt thereof", and $Ar^1$ and $Ar^2$ encompasses various structures.

In contrast, compounds described in the examples of the specification of the present application are mere compounds of formula (I) where
$Ar^1$ is a benzene ring, a pyrimidine ring, a pyridazine ring, a pyrimidine ring, a pyrazole ring, an imidazole ring, a thiophene ring;
$Ar^2$ is a pyrazolyl group, a triazole group, an imidazole group, an oxadiazole group, a pyridyl group, a benzoimidazolyl group, a benzoxazolyl group, a pyrimidinyl group, and a thiazolyl group, and
a substituent on the "heteroaryl group" in $Ar^2$ is unsubstituted or a halogeno group, a C1-C6 alkyl group, a C1-C6 haloalkyl group, a C1-C6 alkoxycarbonyl group, a halogeno group-substituted phenyl group, a C1-C6 haloalkyl group-substituted phenyl group, a C1-C6 alkylaminocarbonyl group, a thiophenyl group, CN, $NH_2$, or $SOCF_3$.

However, generally, properties and usefulness of the compound largely differ depending on the kind and number of elements constituting the compound, the type of bonding between the elements, and the like and are difficult to be predicted by a person skilled in the art on the basis of mere chemical structure, and are not turned out until actual synthesis and actual experiment are performed. Thus, it is not conceivable that all of the groups of compounds encompassed in formula (I) which is different in skeleton structure from good compounds described in the examples have the same properties and usefulness described in the examples even in light of the common general technical knowledge at the time of filing the present application.

The basis for expanding to the entire scope of claim 1 thus cannot be found

The same applies to claims 2-7.

The specification of the present application therefore is not written in such clear and full terms as to enable a person skilled in the art to work the invention in claims 1-7 (PCT Article 5) and does not fully support the invention in claims 1-7 (PCT Article 6).

Therefore, the present search was performed by limiting the compound to the compound represented by formula (I) where

$Ar^1$ is a benzene ring, a pyrimidine ring, a pyridazine ring, a pyrimidine ring, a pyrazole ring, an imidazole ring, a thiophene ring;
$Ar^2$ is a pyrazolyl group, a triazole group, an imidazole group, an oxadiazole group, a pyridyl group, a benzoimidazolyl group, a benzoxazolyl group, a pyrimidinyl group, and a thiazolyl group, and
a substituent on the "heteroaryl group" in $Ar^2$ is unsubstituted or a halogeno group, a C1-C6 alkyl group, a C1-C6 haloalkyl group, a C1-C6 alkoxycarbonyl group, a halogeno group-substituted phenyl group, a C1-C6 haloalkyl group-substituted phenyl group, a C1-C6 alkylaminocarbonyl group, a thiophenyl group, CN, $NH_2$, or $SOCF_3$.

**INTERNATIONAL SEARCH REPORT**
Information on patent family members

International application No.
PCT/JP2019/014789

(Continuation of Box No. A)

CLASSIFICATION OF SUBJECT MATTER
C07D231/12(2006.01)i, A01N41/06(2006.01)i, A01N43/56(2006.01)i, A01N47/04(2006.01)i, A01P5/00(2006.01)i, A01P7/04(2006.01)i, A61K31/415(2006.01)i, A61K31/4184(2006.01)i, A61K31/423(2006.01)i, A61K31/4245(2006.01)i, A61K31/4439(2006.01)i, A61P33/00(2006.01)i, C07D235/18(2006.01)i, C07D249/08(2006.01)i, C07D263/57(2006.01)i, C07D271/06(2006.01)i, C07D271/10(2006.01)i, C07D401/04(2006.01)i, C07D403/04(2006.01)i, C07D413/04(2006.01)i

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2018073976 A
- WO 2006034333 A1
- WO 2005090314 A1

### Non-patent literature cited in the description

- **COLBY, S.R.** Calculating Synergistic and Antagonistic Responses of Herbicide Combinations. *Weeds,* 1967, vol. 15, 20-22